# EUROPEAN PATENT SPECIFICATION

(11) **EP 2 164 501 B1**
(45) Date of publication and mention of the grant of the patent: **20.04.2016**
(21) Application number: 08748030.7
(22) Date of filing: 30.05.2008
(51) Int. Cl.: A61K 31/737, A61P 11/00, A61P 11/06

(54) **SULPHATED XYLANS FOR TREATMENT OR PROPHYLAXIS OF RESPIRATORY DISEASES**
SULFIERTE XYLANE ZUR BEHANDLUNG ODER PROPHYLAXE VON ATEMWEGSERKRANKUNGEN
XYLANES SULFATÉS POUR LE TRAITEMENT OU LA PROPHYLAXIE DE MALADIES RESPIRATOIRES

(30) Priority: 31.05.2007 AU 2007902930 P
(43) Date of publication of application: 24.03.2010
(73) Proprietor: Paradigm Biopharmaceuticals Limited, Melbourne, VIC (AU)
(72) Inventor: COOMBE, Deirdre, Roma, Wembly Downs, Western Australia 6019 (AU); KETT, Warren, Charles, West Lebanon, NH 03784 (US)
(74) Representative: Hoffmann Eitle
(86) International application number: PCT/AU2008/000774
(87) International publication number: WO 2008/144836

(56) References cited:
- WO-A1-91/15216
- WO-A1-99/06025
- WO-A2-2006/000814
- US-A1- 2003 109 491
- US-A1- 2005 282 775
- RAO N V ET AL: "SULFATED POLYSACCHARIDES PREVENT HUMAN LEUKOCYTE ELASTASE-INDUCED ACUTE LUNG INJURY AND EMPHYSEMA IN HAMSTERS" THE AMERICAN REVIEW OF RESPIRATORY DISEASE, AMERICAN THORACIC SOCIETY, US, vol. 142, no. 2, 1 August 1990 (1990-08-01), pages 407-412, XP001041125 ISSN: 0003-0805
- ZENG DEWAN ET AL: "Heparin attenuates symptoms and mast cell degranulation induced by AMP nasal provocation" JOURNAL OF ALLERGY AND CLINICAL IMMUNOLOGY, vol. 114, no. 2, August 2004 (2004-08), pages 316-320, XP002584378 ISSN: 0091-6749
- SOUZA-FERNANDES ALBA B ET AL: "Bench-to-bedside review: the role of glycosaminoglycans in respiratory disease." CRITICAL CARE (LONDON, ENGLAND) 2006 LNKD- PUBMED:17118216, vol. 10, no. 6, 2006, page 237, XP002584379 ISSN: 1466-609X
- WELLSTEIN A. ET AL.: 'Inhibition of fibroblast growth factors' BREAST CANCER RESEARCH AND TREATMENT vol. 38, 1996, pages 109 - 119, XP002129528
- SCHUMACHER K.A. ET AL.: 'The Preventive Effect of a Low-Molecular Pentosan-Polysulphate on DAS-induced increase in Pulmonary Vascular Resistance caused by Platelet-Aggregation' DRUG RES. vol. 23, no. 3, 1973, pages 431 - 433, XP009134014
- JOFFE S.: 'Drug Prevention of Postoperative Deep Vein Thrombosis' ARCH. SURG. vol. 111, January 1976, pages 37 - 40, XP009133964
- TSUBURA E. ET AL.: 'Effects of Sulfated Polysaccharides on Blood-born Pulmonary Metastasis in Rats' GANN vol. 67, December 1976, pages 849 - 856, XP008126481
- KAMITAKAHARA H.: 'Sulfated glycofuranans as inhibitors of melanoma lung metastasis' J. WOOD SCI. vol. 48, 2002, pages 204 - 209, XP008126482
- ANDREWS J.L.: 'The interaction of pentosan polysulfate (SP54) with human neutrophil elastase and connective tissue matrix components' CHEM.-BIOL. INTERACTIONS vol. 47, 1983, pages 157 - 173, XP008126483
- CHIANG G. ET AL.: 'Pentosanpolysulphate (Elmiron) is a potent inhibitor of mast cell histamine secretion' ADV. EXP. MED. BIOL. vol. 539, no. 220, 2003, pages 713 - 729, XP009133960
- OHBAYASHI HIROYUKI: "Neutrophil elastase inhibitors as treatment for COPD", EXPERT OPINION ON INVESTIGATIONAL DRUGS, ASHLEY PUBLICATIONS LTD., LONDON, GB, vol. 11, no. 7, 1 July 2002 (2002-07-01), pages 965-980, XP009148446, ISSN: 1354-3784, DOI: 10.1517/13543784.11.7.965
- J A NADEL ET AL: 'ROLE OF NEUTROPHIL ELASTASE IN HYPERSECRETION IN ASTHMA ROLE OF NEUTROPHIL ELASTASE IN HYPERSECRETION IN ASTHMA' EUR RESPIR J, [Online] vol. 13, 01 January 1999, pages 190 - 196, XP055089501 Retrieved from the Internet: <URL:http://erj.ersjournals.com/content/13/ 1/190.full.pdf#page=1&view=FitH> [retrieved on 2013-11-21]

## Description

### FIELD

The present invention relates generally to agents and medicinal protocols useful in the prophylaxis and/or treatment of respiratory diseases or conditions selected from asthma, allergic rhinitis and chronic obstructive pulmonary disease (COPD). More particularly, the present invention relates to a sulfate xylan in the treatment of these respiratory diseases or conditions., wherein the sulfated xylan is pentosan polysulfate (PPS).

### BACKGROUND

Bibliographic details of the publications referred to by author in this specification are collected at the end of the description.

Reference to any prior art in this specification is not, and should not be taken as, an acknowledgment or any form of suggestion that this prior art forms part of the common general knowledge in any country.

Asthma is characterized by inflammation of the air passages resulting in the temporary narrowing of the airways that transport air from the nose and mouth to the lungs. Asthma symptoms can be caused by allergens or irritants that are inhaled into the lungs, resulting in inflamed, clogged and constricted airways. Symptoms include difficulty breathing, wheezing, coughing, tightness in the chest. In severe cases, asthma can be deadly.

Asthma is probably not a single disease, but rather a complex of multiple, separate syndromes that overlap. The following classification is based on the reports of Wenzel. Lancet; 368:804-813, 2006, and Green et al. Curr. Opin. Allergy Immunol. 7:43-50, 2007.
- Allergic asthma: this is the largest asthma phenotype. This is especially true in childhood asthma but probably also in a high proportion of adults with asthma. Individuals presenting with this phenotype usually experience their first symptoms in childhood, but it can present at any age. Family history of asthma and early exposure to allergens are important in the initiation of allergic asthma. In addition to the standard therapies, targeted therapies: immunotherapy or monoclonal antibodies against IgE, have been used successfully. However, not all people with allergic asthma respond to anti-IgE-therapy.
- Occupational asthma: up to 15% of adult onset asthma falls in this group. It has the following subphenotypes: (1) development of an immunologically mediated response to the causal agent, usually a high molecular weight agent - has similarities to allergic asthma through development of IgE antibodies; (2) development of an immunologically mediated response to low high molecular weight triggers and an IgE response is not consistently seen; (3) development of a non-immunological rapid on-set response after exposure to a high concentration of irritant chemicals.
   The airway inflammation is similar in both immunological phenotypes and resembles that of allergic asthma e.g. presence of eosinophils, lymphocytes, mast cells and the thickening of the reticular basement membrane. By contrast the asthma caused by irritant chemicals is quite different and is characterized by fibrosis of the bronchial wall and epithelial denudation and fibrinohaemorrhagic exudates in the submucosa without eosinophilic inflammation. The immunological types of this asthma can continue in the absence of exposure to the casual agent.
- Aspirin-induced asthma: aspirin and other non-steroidal anti-inflammatory drugs are the triggers. It is common in the severe asthma population and is associated with little evidence of atopy, raised leukotrienes and high numbers of eosinophils in both tissue and blood. There is severe rhinosinusitis and nasal polyps and adult onset. This phenotype is poorly responsive to corticosteroids.
- Menses-related asthma: this is not well characterised. It probably only occurs in a small proportion of women but it can be severe.
- Exercise-induced asthma: the mechanisms that trigger this asthma seem to involve acute inflammatory cell (usually mast cell), epithelial and vasoactive responses but the pathogenesis is unclear. Whether exercise-induced asthma represents the development of bronchoconstriction in response to exercise in all asthmatics or whether it occurs in only some is unclear.

Although inflammation is a hallmark of asthma, not all asthma phenotypes have predominately eosinophilic inflammation, although this is the most common and the best studied.
- Eosinophilic asthma: studies have defined an eosinophilic phenotype by sputum or biopsy testing in patients with varying degrees of asthma severity and have demonstrated consistently around 50% of asthmatics have this phenotype. Other studies have suggested that eosinophilic inflammation may be present in a higher proportion of patients than that detected using sputum or biopsy testing. In one study 50% of the patients with severe asthma thought to be non-eosinophilic actually had eosinophilic inflammation, but in the distal lung.
- Neutrophilic asthma: seen most commonly in patients with severe disease. Many patients with neutrophilic inflammation can have concomitant eosinophilic inflammation in tissue biopsies whereas the sputum assessment may show a clear predominance of neutrophils. The association of neutrophils with severe asthma could be caused by treatment with high dose steroids, which have been shown to decrease neutrophil *apoptosis in vitro.* Neutrophilic asthma was associated within increases in IL-8 and neutrophil elastase. Approximately 20% of patients had neutrophilic asthma and a further 8% had both eosinophlic and neutrophilic inflammation (from a study of 93 patients).
- Paucigranulocytic asthma: patients with sputum cell counts in the normal range (~30% of the 93 patients had this subphenotype).

Allergic rhinitis is an allergen-induced upper-airway disease, characterized by hyperreactive airway mucosa and episodes of symptom chronicity with periods of acute exacerbation. Allergic individuals become sensitized to and may develop IgE antibodies against allergens such as pollens, dust mites, animal dander and mould spores. The immediate allergic response to antigen is termed the early phase response. The mediators released during this phase are histamine, kinins, neutral proteases and a variety of cytokines. Activation of mast cells leads to the production of leukotrienes and prostaglandins and together these mediators give rise to the watery rhinorrhoea, sneezing and itching within minutes of allergen exposure. This is followed several hours later by the late-phase response involving infiltration of inflammatory cells and the release of mediators into the nasal mucosa. Symptoms are similar to that of the early phase response but congestion predominates. *(*Walls et al, Med. J. Aust.; 182:28-33, 2005). Allergic rhinitis has been subdivided into "intermittent" and "persistent" disease. Intermittent disease describes a condition whereby symptoms are present less than 4 days per week, or less than 4 weeks at a time. Persistent disease means that symptoms are present for more than 4 days per week and more than 4 week at a time (Pawanker, Curr. Opin. Allergy Clin. Immunol.; 4:1-4, 2004.)

Allergic rhinitis and allergic asthma are diseases that involve an inflammatory response. They have similar underlying etiology and the key cytokines for each disease are the Th2 subset of T-cell cytokines IL-5, IL-4 and IL-13 and GM-CSF. These diseases are characterized by a marked inflammatory cell infiltrate comprising eosinophils, mast cells, T-lymphocytes and cells of the monocytic lineage. The adhesion molecules, P-selectin, MAC-1 and PECAM-1 play an important role in the extravasation of leukocytes and are likely to be involved in the inflammatory process. Further, the eotaxin family of chemokines plays a key role in these diseases as they are the prime chemotactic factors stimulating eosinophil and CD4 + T lymphocyte infiltration.

There is a growing realization that asthma and allergic rhinitis are components of a single inflammatory airway disease. A conclusion that is supported by epidemiological data showing that more than 80% of persons with allergic asthma have allergic rhinitis, and that up to 50% of patients with allergic rhinitis have asthma (Gelfand, J. Allergy Clin. Immunol , 114:S135-138, 2004; Passalacqua et al, Curr. Opin. Allergy Clin. Immunol. 4:177-183, 2004). Moreover, longitudinal and follow-up studies have shown that rhinitis usually precedes asthma and is a risk factor for asthma. Allergic Rhinitis increases the risk of developing asthma by at least three-fold and correct treatment of allergic rhinitis with intranasal steroids has a favourable effect on bronchial symptoms, significantly reducing the rate of hospital admittance and emergency department visits for asthma exacerbation (Passalacqua et al., Curr. Opin. Allergy Clin. Immunol. 4:177-183, 2004). These diseases are a complex mixture of pathologies, involving at least the various cytokines, chemokines and cell adhesion molecules indicated above.

Mast cells and histamines play an important role during the initial allergic rhinitis response. However, as allergic rhinitis progresses the role of histamines diminishes, making anti-histamines less effective as a therapy (Gelfand, J. Allergy Clin. Immunol 114: S135-138, 2004). During the initial allergic rhinitis response sensitized mast cells degranulate within minutes of allergen exposure releasing preformed and newly synthesized mediators including histamine, proteases, cysteinyl leukotrienes, prostaglandins and cytokines. Allergic rhinitis progression is dependent upon mediators associated with the infiltration of eosinophils, basophils, neutrophils, mononuclear cells and T-lymphocytes *(Passalacqua et al, supra* 2004.). The association of eosinophils and IL-5 with allergic rhinitis has been appreciated for some time. Repeated studies have found increased levels of Th2-type cytokines including IL-5 and IL-4, and increased amounts of eosinophil cationic protein (ECP), a marker of activated eosinophils, following provocation with allergen (Blaiss Allergy Asthma Proc. 26: 35-40, 2005. The influx of eosinophils correlates closely with the development of symptoms. In addition the loss of epithelial integrity in the nasal mucosa of rhinitis patients correlates with eosinophil numbers rather than the numbers of mast cells or neutrophils (Borish J. Allergy Clin Immunol. 112: 1021-1031, 2003). It seems allergic rhinitis evolves from an acute, primarily mast cell-mediated process that is responsive to anti-histamines, through to a chronic inflammatory disease that is primarily eosinophil-mediated and is much less responsive to anti-histamines. This is the case for patients with persistent allergic rhinitis. Progression to a condition that is refractory to anti-histamines can also occur within an allergy season, for seasonal suffers. For other patients with mild intermittent disease antihistamines do remain an effective therapy, reflecting intermittent allergen exposures, which are not of sufficient duration to drive disease progression into the anti-histamine-resistant phase.

The first line treatment for asthma is inhaled corticosteroids (ICS), which are usually used in combination with β₂-agonists (Barnes, Br. J. Pharmacol. 147 Suppl 1:S297-303, 2006). β₂-agonists relieve the symptoms rather than treat the underlying inflammation and have the potential to make asthma worse if used frequently in the absence of ICSs. Nevertheless, this seems to be the therapy preferred (despite its side-effects) because of the immediacy of its effect. β₂-agonist therapy in the absence of ICS has been given a "black box" listing by the FDA because of the potential cardiac problems associated with this therapy. Although side effects are lower than with oral formulations ICS are not without adverse local and systemic side effects. A side effect of corticosteroids is the suppression of the hypothalamic-pituitary-adrenal axis (HPAA): clinically relevant adverse effects are seen and this is more apparent with some medications than others. Bone density and fractures can also be a problem: certain effects of ICS on bone metabolism are detectable but the clinical relevance is unclear. Finally growth retardation in children is another issue that may worry patients (Allen, Adv Pediatr. 53:101-110, 2006). On balance the side effects are acceptable given the severe complications of sustained/uncontrolled asthma.

Other therapies for asthma include:
(1) Omalizumab, an anti-IgE antibody (Genetech) and is also viewed as not cost effective for standard asthma treatment. It is primarily used as add-on therapy to ICS because it does not improve airway responsiveness and has modest efficacy. The dose constraints and delivery mechanism (subcutaneous injection) are an added disadvantage. Moreover, a warning from the US Food and Drug Administration (FDA) has linked omalizumab injection to life-threatening anaphylaxis and more worrying in some patients this anaphylaxis is delayed occurring more than 2 hours after injection to more than 24 hours after injection.
(2) Anti-leukotrienes (anti-LTs), which can cause bronchodilation. Their effect is additive to that of short-acting β₂-receptor agonists although alone they have a relatively modest effect. Anti-LTs primarily affect the early asthmatic response (EAR) whereas, ICS show pronounced effects on late asthmatic responses *(*Palmqvist et al, Allergy 60:65, 2005). For these reasons anti-LTs have been trialed in combination with ICS. Anti-leukotrienes are not cost effective. They have virtually no side effects, but their efficacy is low.

The usual therapies for allergic rhinitis are anti-histamines or intranasal corticosteroids Neilsen and Dahl, Am. J. Respir. Med. 2:55-65, 2003; Yanez and Rodrigo, Ann. Allergy Asthma Immunol. 89:479-84, 2002). The older first generation oral H1 antagonists (anti-histamines) have a number of adverse side-effects, the best recognized being drowsiness and anticholinergic effects. The second generation drugs were developed to overcome these effects. However, recent studies have indicated that the division between first and second generation H1 antagonists in terms of drowsiness is not that clear cut (Golightly and Greos, Drugs 65:341-84, 2005). Labels for Cetirizine, the most potent anti-histamine approved by the FDA, include a warning about the possible adverse effect of somnolence and caution with driving and use of heavy equipment when taking the drug was urged. In addition, concurrent use of alcohol or other central nervous system (CNS) suppressants should be avoided because additional reduction in alertness and CNS performance may occur. Moreover, anti-histamines are not effective against the congestion associated with chronic allergic rhinitis. Allergic rhinitis progresses to a disease that is primarily eosinophil mediated and refractory to anti-histamine therapy. When this happens intranasal corticosteroids (INCS) are the main therapy. Indeed, for many clinicians INCS are the drugs of choice for treatment of all allergic rhinitis as the corticosteroid acts to reduce eosinophil inflammation. Although INCS are generally considered safe the recommendation is to reduce steroid dose as much as possible and to optimize steroid-sparing strategies (Skoner, Curr. Opin. Allergy Clin. Immunol. 2:7-10, 2002).

The underlying etiology of chronic obstructive pulmonary disease (COPD) is different from that of allergic inflammatory diseases (Sutherland and Martin, J Allergy Clin. Immunol. 112:819-27, 2003). COPD involves a chronic inflammatory process affecting peripheral airways and lung parenchyma and inflammation is worse during exacerbations. A major contributory factor to the development of COPD is the inflammatory response to cigarette smoke. The pathological indicators of COPD are destruction of the lung parenchyma (pulmonary emphysema), inflammation of the small peripheral airways (respiratory bronchitis) and inflammation of the central airways. Most patients with COPD have all three pathological conditions (chronic obstructive bronchitis, emphysema and mucus plugging) that exhibit different patterns of inflammation (Adcock and Ito, Proc. Am. Thorac. Soc. 2:313-319, 2005). Neutrophils and macrophages are considered to be the main effectors of disease. Analyses of sputum and bronchoalveolar lavage fluid show increases in both neutrophils and macrophages in these secretions from COPD patients. In addition, there is increasing evidence that a significant sub-group of COPD patients exist who have chronic airway eosinophilia.

Alveolar macrophages play a key role in COPD, they are localized to sites of alveolar destruction, and their numbers are positively correlated with disease severity, airway obstruction and degree of alveolar wall damage in emphysema. Airway tissue neutrophils are increased in the large and small airways of COPD patients during exacerbations and in severe COPD, or during infections. Patients with COPD also display either an increase in the CD8+/CD4+ T cell ratio, or an increase in the total numbers of both CD8+ and CD4+ T cells in the airway wall (MacNee, Proc. Am. Thorac. Soc. 2:258-266, 2005). The bronchioles are obstructed by fibrosis and infiltrated with macrophages and T lymphocytes.

There are three morphological forms of COPD: chronic bronchitis, obstructive bronchiolitis and emphysema (Szilasi et al., 2006. Pathol. Oncol. Res. 12:52-60). The inflammation associated with chronic bronchitis is located in the epithelium of the central airways. The inflammatory process is associated with increased production of mucus and defective mucociliary clearance. Inflammation is observed in the mucosa, in the smooth muscle layers and submucosal glands. In large airways mononuclear cell, macrophage, CD8+ T cells and plasma cell involvement is common in stable COPD and during exacerbations of chronic bronchitis. CD8+ T cells release tumor necrosis factor-α (TNF-α), a potent proinflammatory mediator. The role of the neutrophil is not clear. Neutrophils are seen in the large airways only during exacerbations and in severe COPD, they are however, observed early on in the airway lumen and in the sputum.

Obstructive bronchiolitis or small airway obstruction is an inflammatory condition that involves the small and peripheral airways. The typical feature is collapsed lumen with increased mucus. Macrophages and CD8+ T cells dominate small airway inflammation, although the inflammatory changes showed a positive correlation with airflow obstruction in COPD. For example, in mild to moderate stable COPD macrophages were dominant, while in severe disease neutrophils were the predominant inflammatory component, whilst during mild exacerbations eosinophils are found. Increased numbers of fibrobalsts and myofibroblasts and enhanced extracellular matrix is found in the subepithelium of the small airways in obstructive bronchiolitis. This pathology suggests a mechanism of repetitive injury and healing that leads to fibrosis and scar tissue. The net result is airway narrowing.

Emphysema is defined by permanent air space enlargement caused by destruction and enlargement of lung tissue beyond the terminal bronchiole. The mechanism of the disease involves unregulated inflammation and the release of large amounts of proteolytic enzymes. Protease/antiprotease imbalance is the presumed cause for pulmonary emphysema. Although inflammation is dominated by CD8+ T cells, macrophages and neutrophils produce excessive amounts of proteases including leukocyte elastase, cathepsin G, proteinase 3, matrix metalloprotineases (MMPs), cystein proteinases and plasminogen activator. These enzymes destroy the elastin and other components of the alveolar wall with elastase being the enzyme most heavily implicated in this process.

The proinflammatory mediators of these disease processes include leukotriene- B4, IL-8 and other chemokines (e.g. MIP-1α, MCP-1), TNF-α, IL-13 and IL-4 (Barnes, Pharmacol. Rev. 56:515-548, 2004). It has been suggested that the inhibitory effects of TNF-α and IL-4 on the production of the regulatory cytokine TGF-β by bronchial epithelial cells may contribute to the progression of the inflammatory response. In addition, increased levels of IL-6, IL-1β, TNF-α, and IL-8 have been measured in sputum with further increases during exacerbations.

COPD is a very significant burden on society. It is the fifth leading cause of death in the UK. It affects 5% of the adult population and is the only major cause of death in the US in which morbidity and mortality are increasing. By 2020 it is estimated that COPD will be the 3^{rd}-leading cause of death and the 5^{th}-leading cause of disability worldwide (Halpin and Miravitlles, Proc. Am. Thorac. Soc. 3:619-623, 2006). Existing therapies for COPD are grossly inadequate. None slow disease progression and response to treatments is poor. COPD is relatively resistant to the anti-inflammatory effects of corticosteroids. Nevertheless current pharmacologic options include drugs to assist in stopping smoking, short and long-acting β₂-agonists, short and long acting anticholinergics, inhaled corticosteroids, theophylline, N-acetyl cysteine and other mucolytics and oxygen (Anzueto, Am. J. Med. 119:S46-S53, 2006; Barnes and Stockley, Eur. Respir. J. 25:1084-1106, 2005). The short and long-acting β₂-agonists were introduced to improve bronchodilation. They are often used in combination with anticholinergics because they produce bronchodilation *via* different pathways. Inhaled corticosteroids are often used in combination with β₂-agonists and improvements in exacerbation rates are greater than that seen with the individual component. Theophylline is a useful bronchodilator. The mode of action of N-acetyl cysteine is not clear but it may act as a mucolytic or antioxidant to improve cough symptoms and in some patients appear to reduce exacerbation frequency.

Despite these therapies the only intervention clearly shown to reduce mortality in clinical trials is smoking cessation.

### SUMMARY

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Pentosan polysulfate (PPS) and other sulfated xylan-like compounds are identified as being useful in the treatment and prophylaxis of respiratory conditions selected from asthma, allergic rhinitis and congestive obstructive pulmonary disease (COPD). Reference to "COPD" includes emphysema, chronic bronchitis and obstructive bronchiolitis. Whilst PPS has been suggested for use in the treatment of deep vein thrombosis, it has now been determined that sulfated xylans, i.e. PPS, are useful in the prevention of, or in the amelioration of symptoms for, respiratory disease. Hence, sulfated xylans, i.e. PPS, are proposed for use in the treatment of a respiratory condition selected from the list of asthma, allergic rhinitis, COPD. The term "respiratory disease" or "respiratory condition" includes a pulmonary disease or condition such as an inflammatory and/or allergic respiratory or pulmonary conditions.

Sulfated xylans (e.g. PPS), may be synthetic or semi-synthetic in origin and/or may be generated from larger sulfated polysaccharides. In addition, the compounds described herein may undergo a number of other modifications such as the addition of side branches, sulfation and/or phosphorylation of the xylan backbone and changes in length of the polysaccharide chain. The agents may also be fused to or be part of amino acid moieties or peptide, polypeptide or protein chains, referred to herein as "composite" molecules.

Accordingly, a method is disclosed for the treatment or prophylaxis of a respiratory condition in a subject, the method comprising administering to the subject an effective amount of a sulfated xylan or a derivative or homolog thereof or a salt thereof for a time and under conditions sufficient for amelioration of symptoms of the respiratory condition.

The sulfated xylan is pentosan polysulfate (PPS).

Hence, a method is further disclosed for the treatment or prophylaxis of a respiratory condition in a subject, the method comprising administering to the subject an effective amount of a PPS or a functional derivative or homolog thereof or a salt thereof for a time and under conditions sufficient for amelioration of symptoms of the respiratory condition.

In an embodiment, the medicament is administered *via* inhalation or as a nasal spray.

A "functional" derivative or homolog of a sulfated xylan such as PPS as disclosed herein is a compound, which ameliorates the symptoms being treated or prevented in a manner similar to the sulfated xylan. A derivative or homolog also includes a composite molecule comprising a xylan sulfate and an amino acid moiety or a peptide, polypeptide or protein. A composite molecule may also comprise multiple sulfated xylan molecules and/or multiple amino acids or multiple peptide, polypeptide or protein entities.

The respiratory condition is selected from the list comprising asthma, allergic rhinitis and COPD, including emphysema, chronic bronchitis and obstructive bronchiolitis.

Hence, this aspect is directed to a pharmaceutical composition comprising PPS for the treatment or prophylaxis of a respiratory condition selected from the list of asthma, allergic rhinitis and COPD (including emphysema, chronic bronchitis and obstructive bronchiolitis) comprising administering to the subject a sulfated xylan or a derivative or homolog thereof are a salt thereof for a time and under conditions sufficient for amelioration of symptoms of the respiratory condition.

In a particular aspect, the purpose of treatment or prophylaxis of a respiratory condition selected from the list of asthma, allergic rhinitis and COPD (including emphysema, chronic bronchitis and obstructive bronchiolitis) is contemplated, the purpose being administering to said subject PPS for a time and under conditions sufficient for amelioration of symptoms of the respiratory condition.

Still another aspect is directed to the use of a sulfated xylan, i.e. PPS, in the manufacture of a medicament for the treatment or prophylaxis of a respiratory condition or disease. Respiratory conditions and diseases contemplated herein are asthma, allergic rhinitis and COPD (including emphysema, chronic bronchitis and obstructive bronchiolitis).

Compositions comprising sulfated xylans or their derivatives or homologs for the treatment of respiratory conditions selected from asthma, allergic rhinitis and COPD (including emphysema, chronic bronchitis and obstructive bronchiolitis) are also contemplated herein.

Yet another aspect herein discloses medicinal protocols for treating or preventing respiratory conditions. The protocols include diagnosis of a respiratory condition or disease in a subject, prescription of a type and amount of sulfated xylan such as PPS or a functional derivative or homolog thereof and monitoring of the subject for amelioration of symptoms and/or other signs of the disease condition. The protocols may also include prescription of other active agents.

In accordance with the above aspects, a subject may be any animal, such as a mammal and in particular a human.

Although not intending to limit the present invention to any one theory or proposed mode of action, the xylan sulfate may act as an antagonist of an inflammatory molecule such as a cytokine or growth factor amongst other molecules. Hence, the present specification also discloses a ligand to which the sulfated xylan such as PPS binds. Hence, a fraction or derivative of the sulfated xylan such as a PPS or a fraction or derivative thereof may act as an antagonist of a ligand. Examples of ligands include, but are not limited to: a cytokine including an interleukin (e.g. IL-5, IL-4, IL-13, IL-6, IL-7, IL-8, IL-9, T-10, IL-11, IL-12, IL-3, IL-14, IL-15 or IL-17 family, of cytokines including IL-25), interferon (e.g. α-interferon, β-interferon, γ-interferon) or a growth factor including but not limited to G-CSF, M-CSF, GM-CSF, BDNF, CNTF, EGF, EPO, FGF1, FGF2, FGF3, FGF4, FGF5, FGF6, FGF7, FGF8, FGF9, FGF10, FGF11, FGF12, FGF12, FGF13, FGF14, FGF15, FGF16, FGF17, FGF18, FGF19, FGF20, FGF21, FGF22, FGF23, LIF, PDGF, SCF, TGFα, TGFβ, TNFα, TNFβ, TPO, VEGF, GH, NGF, NT 3, NT4, NT5, NT6, NT7, oncostatin M (OSM) and insulin, or a chemokine including but not limited to MCP-1, MCP-2, MCP-3, MCP-4, MCP-5, a member of the MIP-1 family including MIP-1α, MIP-2, eotaxin (eotaxin-1, -2 or-3), PBP (platelet basic protein), SDF-1, PBSF, PF4, RANTES, and the like; an enzyme such as elastase; an enzyme of the cathepsin family, cell adhesion molecules such as PECAM-1; or a soluble or cell- or virus-bound receptor.

Accordingly, the present specification discloses a composition comprising a fraction or derivative of a sulfated xylan which is an antagonist of a ligand selected from the list comprising IL-5, IL-4, IL-13, IL-6, IL-7, IL-8, IL-9, IL-10, IL-11, IL-12, IL-3, IL-14, IL-15 or IL-17 family of cytokines including IL-25, interferon, G-CSF, M-CSF, GM-CSF, BDNF, CNTF, EGF, EPO, FGF1, FGF2, FGF3, FGF4, FGF5, FGF6, FGF7, FGF8, FGF9, FGF10, FGF11, FGF12, FGF12, FGF13, FGF14, FGF15, FGF16, FGF17, FGF18, FGF19, FGF20, FGF21, FGF22, FGF23, LIF, PDGF, SCF, TGFα, TGFβ, TNFα, TNFβ, TPO, VEGF, GH, NGF, NT 3, NT4, NT5, NT6, NT7, oncostatin M (OSM), insulin, MCP-1, MCP-2, MCP-3, MCP-4, MCP-5, a member of the MIP-1 family including MIP-1α, MIP-2, eotaxin (eotaxin-1, -2 or -3), PBP (platelet basic protein), SDF-1, PBSF, PF4, RANTES, elastase; an enzyme of the cathepsin family, a cell adhesion molecules such as PECAM-1 and a soluble or cell- or virus-bound receptor, said composition further comprising one or more pharmaceutical acceptable carriers, diluents and/or excipients.

Particular ligands are IL-5, IL-4, IL-13, G-CSF and M-CSF; particular ligands also include IL-8, MCP-1, MIP-1α eotaxin-1 and eotaxin-2.

The sulfated xylan may be in a pharmaceutical composition together with one or more pharmaceutically acceptable carriers, diluents and/or excipients.

Abbreviations used herein are defined in Table 1.

**Table 1**

| **Abbreviations** | |
|---|---|
| **Abbreviation** | **Definition** |
| Anti-LT | Anti-leukotrienes |
| AR | Allergic rhinitis |
| BALF | Bronchoalveolar lavage fluid |
| COPD | Chronic obstructive pulmonary disease |
| D.P. | Degree of polymerization |
| GAG | Glycosaminoglycan |
| GlcA | Glucuronic acid |
| GlcN | Glucosamine |
| h | Human |
| H | Heparin |
| HL | Heparin-like |
| HLGAG | Heparin-like glycosaminoglycan |
| HPAA | Hypothalamic pituitary adrenal axis |
| ICS | Inhaled corticosteroids |
| IdoA | Iduronic acid |
| INCS | Intranasal corticosteroids |
| LT | Leukotrienes |
| MMP | Matrix metalloproteinase |
| OVA | Ovalbumin |
| PPS | Pentosan polysulfate |
| r | Recombinant |

### BRIEF DESCRIPTION OF THE FIGURES

**Figure 1** is a schematic representation of pentosan polysulfate (PPS).
**Figure 2** is a schematic representation of the sulfated xylo-oligosaccharides, n=1,-9, X=H or SO₃Na. The anomericity of the terminal residue (reducing terminus prior to sulfation) may be α, β or a mixture of both.

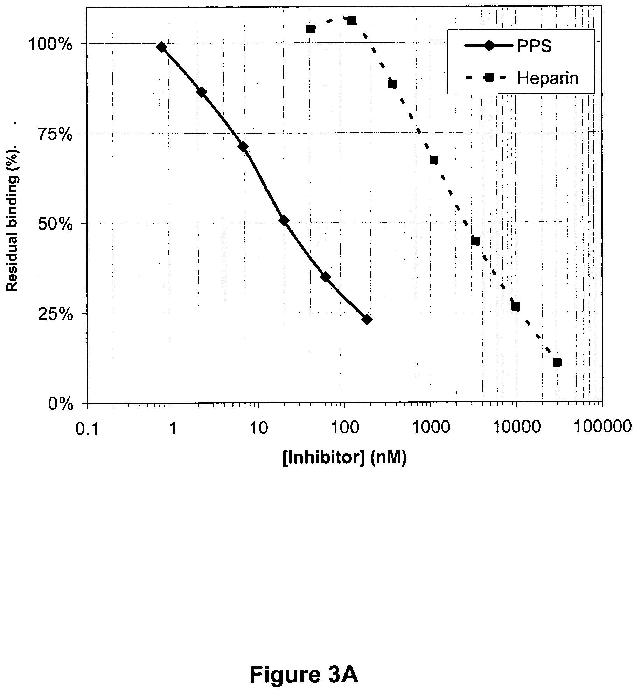

**Figures 3A-E** are graphical representations showing: A) the dose-response curves of pentosan (full line) and heparin (dashed line) for the inhibition of binding of IL-4; B) the dose-response of pentosan (full line) and heparin (dashed line) for the inhibition of binding of IL-5. In each case, binding was measured using a heparinized flow cell on the BIAcore. C) Binding of different concentrations of human eotaxin (CCL11) to heparin immobilized on a sensor surface, sensorgrams generated by a BIAcore 2000 are shown; D) dose-response of pentosan polysulfate (blue diamonds), heparin (green triangles) and a sulfated xylan consisting of 8 saccharides (DP 8) (red squares) for the inhibition of binding of eotaxin-1 to a heparinized flow cell on the BIAcore; E) dose-response of pentosan poly sulfate (red squares) and heparin (blue diamonds) for the inhibition of binding of eotaxin-2 to a heparinized flow cell on the BIAcore.
**Figure 4** is a graphical representation showing the dose-response curves of pentosan (full line) and heparin (dashed line) for IL-5 dependent proliferation of Ba/F-IL-5 cells.
**Figure 5** is a graphical representation showing the dose-response curves of pentosan (full line) and heparin (dashed line) for IL-4 dependent proliferation of TF-1.8 cells.
**Figure 6** is a graphical representation showing the dose-response curve for the GM-CSF dependent proliferation of TF-1.8 cells.
**Figure 7** is a graphical representation showing the effect of sulfated carbohydrates on the proliferation of TF-1.8 cell proliferation obtained with 0.025ng/ml of GM-CSF.
**Figure 8** is a graphical representation showing the dose-response curve for the IL-2 dependent proliferation of CTL-Luc cells.
**Figure 9** is a graphical representation showing the effect of sulfated carbohydrates upon the proliferation of CTL-Luc cells obtained with 1.25ng/ml of recombinant human (rh)IL-2.
**Figure 10** **A and B** are graphical representations showing: A) a dose response curve for the inhibition of the activity of human leukocyte elastase by PPS; and B) a dose response curve for the inhibition of human leukocyte elastase by heparin.
**Figure 11** is a graphical representation showing the effect of both heparin (H) and pentosan polysulfate (PPS) on the protein content of nasal lavage fluid from guinea pigs sensitized with ovalbumin (OVA) and challenged intranasally with OVA (Allergic rhinitis animal model). These data were compared with the positive control that was sensitized and challenged with OVA but not treated with the drugs. Statistical significance is shown (* = P<0.05, ** = P<0.01), and % inhibition is given. Heparin and PPS were used at 1, 5, 10 and 20 mg/kg.

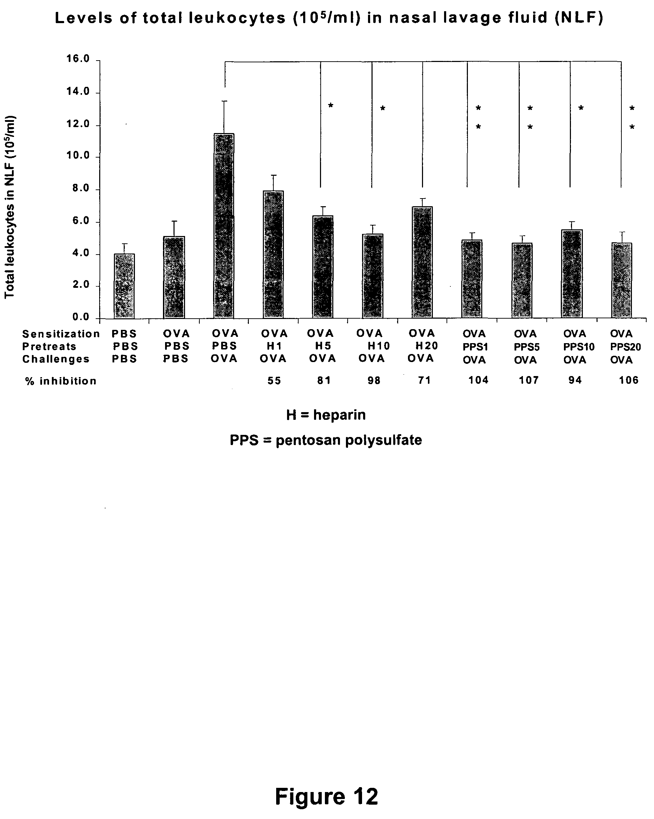

**Figure 12** is a graphical representation showing the effect of both heparin (H) and pentosan polysulfate (PPS) on the levels of leukocytes infiltrating into the nasal cavities and collected in the nasal lavage fluid from guinea pigs sensitized with ovalbumin (OVA) and challenged intranasally with OVA (Allergic rhinitis animal model). These data were compared with the positive control that was sensitized and challenged with OVA but not treated with the drugs. Statistical significance is shown (* = P<0.05, ** = P<0.01), and % inhibition is given. Heparin and PPS were used at 1, 5, 10 and 20 mg/kg.
**Figure 13** is a graphical representation showing the effects of PPS and the sulfated xylan tetra- and penta-saccharide mixture on the cellular infiltrate contained in the nasal lavage fluid of guinea pigs sensitized with ovalbumin (OVA) and challenged intranasally with OVA (allergic rhinitis animal model). Both drugs were used at 5mg/kg. These data were compared with the positive control that was sensitized and challenged with OVA but not treated with the drugs. Statistical significance is shown (* = P<0.05, ** = P<0.01), and % inhibition is given.
**Figure 14** is a graphical representation of PPS inhibiting the level of protein (A) and the leukocyte infiltrate (B) in bronchoalveolar lavage fluid collected from guinea pigs sensitized with ovalbumin (OVA) and challenged with inhaled OVA (asthma model). PPS was used at 0.1, 0.5, 2.5 and 10 mg/kg. Statistical significance is shown (* = P<0.05) and % inhibition is given.
**Figure 15** is a graphical representation of the effect of various concentrations of PPS on components of airway hyperreactivity measured in guinea pigs sensitized with OVA and challenged with inhaled OVA (asthma model). The response to three concentrations of methacholine (3, 10 and 30 mg/ml) are shown. PPS was used at 0.1, 0.5 and 2.5 mg/kg. Statistical significance is shown (** = P<0.01) for a comparison with the positive control (sensitized and challenged with OVA but given PBS). (A) Airway resistance (R_{L}); (B) Lung compliance (C_{Dyn}).
**Figure 16** is a graphical representation of the effect of PPS and the sulfated xylan pentasaccharide used at 20, 10, 2.5 and 1.25 µg/ml on IL-13 (2.5ng/ml) mediated proliferation of TF-1 cells. Shown is % inhibition relative to the positive control, which was IL-13 with no sulfated drugs.
**Figure 17** is a graphical representation of the effect of PPS and the sulfated xylan pentasaccharide on the ability of DMSO treated HL-60 cells to migrate in response to 20ng/ml of IL-8. The number of cells migrating into the bottom chamber of a 96-well chemotaxis plate was quantified using AQUEOUS ONE dye with absorbance being measured at 490 nm. PPS and the sulfated xylan pentasaccharide were used at 10 and 50 µg/ml. Cell migration in the absence of chemokine (no agent) and with IL-8 but no inhibitor are shown.
**Figure 18** is a graphical representation of the effect of PPS and the sulfated xylan pentasaccharide on the ability of THP-1 cells to migrate in response to 10ng/ml of MCP-1. The number of cells migrating into the bottom chamber of a 96-well chemotaxis plate was quantified using AQUEOUS ONE dye with absorbance being measured at 490 nm. PPS and the sulfated xylan pentasaccharide were used at 10 and 50 µg/ml. Cell migration in the absence of chemokine (no agent) and with MCP-1 but no inhibitor are shown.
**Figure 19** is a graphical representation of the effect of PPS and the sulfated xylan pentasaccharide on the ability of DMSO treated U937 cells to migrate in response to 40ng/ml of MIP-1α. The number of cells migrating into the bottom chamber of a 96-well chemotaxis plate was quantified using AQUEOUS ONE dye with absorbance being measured at 490 nm. PPS and the sulfated xylan pentasaccharide were used at 10 and 50 µg/ml. Cell migration in the absence of chemokine (no agent) and with MIP-1α but no inhibitor are shown.

### DETAILED DESCRIPTION

The present invention is defined by the claims.

This specification discloses the following aspects.

Unless otherwise indicated, the subject specification is not limited to specific formulations of components, manufacturing methods, dosage regimens or the like, as such may vary. Terminology used herein is for the purpose of describing particular embodiments or aspects only and is not intended to be limiting.

As used herein, the singular forms "a", "an" and "the" include plural aspects unless the context clearly dictates otherwise. Thus, for example, reference to "a sulfated xylan" includes a single sulfated xylan, as well as two or more sulfated xylans; reference to "an active agent" includes a single active agent, as well as two or more active agents; reference to "the invention" includes a single aspect or multiple aspects of an invention; and so forth.

The terms "compound", "active agent", "chemical agent", "pharmacologically active agent", "medicament", "active", "drug" and the like are used interchangeably herein to refer to a chemical compound such as a sulfated xylan that induces a desired pharmacological and/or physiological effect. In this context, the effect includes the treatment or prophylaxis of or the amelioration of symptoms associated with a respiratory condition such as asthma, allergic rhinitis and/or COPD (including emphysema, chronic bronchitis and obstructive bronchiolitis). The terms also encompass pharmaceutically acceptable and pharmacologically active ingredients of those active agents specifically mentioned herein including but not limited to salts, esters, amides, prodrugs, active metabolites, analogs and the like. When the terms "compound", "active agent", "chemical agent" "pharmacologically active agent", "medicament", "active", "drug" and the like are used, then it is to be understood that this includes the active agent *per se* as well as pharmaceutically acceptable, pharmacologically active salts, esters, amides, prodrugs, metabolites, analogs, etc. Composite molecules and compositions are also encompassed.

Reference to a "compound", "active agent", "chemical agent" "pharmacologically active agent", "medicament", "active", "drug" and the like includes combinations of two or more actives such as two or more sulfated xylans or sulfated xylan-composite molecules or a sulfated xylan and another therapeutic agent. A "combination" also includes multi-part such as a two-part pharmaceutical composition where the agents are provided separately and given or dispensed separately or admixed together prior to dispensation. For example, a multi-part pharmaceutical pack may have a sulfated xylan or population of sulfated xylans and/or one or more anti-inflammatory agents. An "anti-inflammatory agent" includes an anti-histamine as well as inhibitors of a colony stimulating factor (CSF) or growth factor or cytokine such as an antagonist of IL-5, IL-4" IL-13, G-CSF or M-CSF or inhibitors of a chemokine such as IL-8, MCP-1, a member of the MIP-1 family e.g. MIP-1α, or eotaxin or a receptor thereto, or an enzyme like leukocyte elastase,. Examples of antagonists include antibodies and soluble receptors.

Reference to a list of cytokines such as " IL-5, IL-4, IL-13, G-CSF or M-CSF" or chemokines such as "IL-8, MCP-1, eotaxin (eotaxin-1, -2 or -3), a member of the MIP-1 family, e.g. MIP-1α " means that in one embodiment, the cytokine is IL-5; in another embodiment it is IL-4; in another embodiment it is IL-13; in another embodiment it is IL-8; in another embodiment it is MPC-1; in another embodiment it is MIP-1α; and in another embodiment it is G-CSF, and in another embodiment it is M-CSF.

The terms "effective amount" and "therapeutically effective amount" of an agent as used herein mean a sufficient amount of the agent to provide the desired therapeutic or physiological effect. Undesirable effects, e.g. side effects, are sometimes manifested along with the desired therapeutic effect; hence, a practitioner balances the potential benefits against the potential risks in determining what is an appropriate "effective amount". The exact amount required varies from subject to subject, depending on the species, age and general condition of the subject, mode of administration and the like. Thus, it may not be possible to specify an exact "effective amount". However, an appropriate "effective amount" in any individual case may be determined by one of ordinary skill in the art using routine experimentation. The effective amount in this context includes an amount required to treat or prevent or ameliorate the symptoms of a respiratory condition such as asthma, allergic rhinitis and/or COPD (including emphysema, chronic bronchitis and obstructive bronchiolitis). By "ameliorate" is included relieving of adverse symptoms, inducing a state of comfort or wellbeing or removing or reducing biochemical, physiological or clinical markers of the disease.

By "pharmaceutically acceptable" carrier, excipient or diluent is meant a pharmaceutical vehicle comprised of a material that is not biologically or otherwise undesirable, i.e. the material may be administered to a subject along with the selected active agent without causing any or a substantial adverse reaction. Carriers may include excipients and other additives such as diluents, detergents, coloring agents, wetting or emulsifying agents, pH buffering agents, preservatives and the like. A composition may also be described *inter alia* as a medicament, formulation, agent or drug.

Similarly, a "pharmacologically acceptable" salt, ester, amide, prodrug or derivative of a compound as provided herein is a salt, ester, amide, prodrug or derivative that this not biologically or otherwise undesirable.

The terms "treating" and "treatment" as used herein refer to reduction in severity and/or frequency of symptoms of the disease condition or infection, elimination of symptoms and/or underlying cause, prevention of the occurrence of symptoms of the disease condition or infection and/or their underlying cause and improvement or remediation of damage following a disease or condition. The condition being treated or prevented is a respiratory condition. The terms "respiratory condition" and "respiratory disease" may be used interchangeably herein. In one aspect, the respiratory disease or condition is a pulmonary disease or condition. In another aspect, the respiratory or pulmonary disease or condition is an inflammatory and/or allergic disease or condition.

"Treating" a patient may involve prevention of a disease or condition or adverse physiological event in a susceptible individual as well as treatment of a clinically symptomatic individual by inhibiting a disease or condition.

"Patient" as used herein refers to an animal, such as mammal including a human who can benefit from the pharmaceutical formulations and methods described herein. There is no limitation on the type of animal that could benefit from the presently described pharmaceutical formulations and methods. A patient regardless of whether a human or non-human animal may be referred to as an individual, subject, animal, host or recipient as well as patient. The compounds and methods described herein have applications in human medicine, veterinary medicine as well as in general, domestic or wild animal husbandry as well as the racing industry in relation to respiratory conditions.

Polyanions have been suggested as treatments for asthma and allergic rhinitis, including heparin and low-molecular weights heparins, modified heparins, oligosaccharides prepared from heparin, sulfated maltohexaose and sulfated saccharides of size D.P. 2-4 *[*Suchankova et al, Eur J Pharmacol, 507(1-3):261-71, 2005. Effects of bemiparin on airway responses to antigen in sensitized Brown-Norway rats; Parish et al, US Patent 6,143,730; Kuszmann et al, WO2006017752]. Significantly, the molecules described in these references have a higher degree of sulfation per monosaccharide residue than those of the present invention. Xylans are limited to a maximum of two sulfates per residue, whilst oligosaccharides containing glucose can possess three sulfates per residue. Heparins and low molecular weight heparin preparations suffer from the drawback that they are anticoagulants and hence compromise the safety of medicaments for routine use in asthma and allergic rhinitis. In contrast, PPS is cheap to manufacture and readily available commercially in a suitable form for administration. Moreover, due to its long term use as a therapeutic the pharmacology of PPS is well established. In particular, because it is a weak anticoagulant with only about 1/15^{th} of the anticoagulant of heparin, it is less likely to have the safety concerns that accompany the use of heparin as a medicament for treating respiratory diseases. Pentosan polysulfate has been previously used as a thromboprophylactic to prevent deep vein thrombosis. It has also been investigated for use in prion disease, as an anti-angiogenic agent in oncology, as a reagent to block HIV-1 infection, a reagent to treat inflammatory bowel disease and a treatment for rheumatoid arthritis. It has also been used in veterinary medicine to treat non-infectious joint disease particularly in dogs and horses, and more particularly, osteoarthritis, traumatic joint and peri-articular inflammation, and osteochondrosis dissecans.

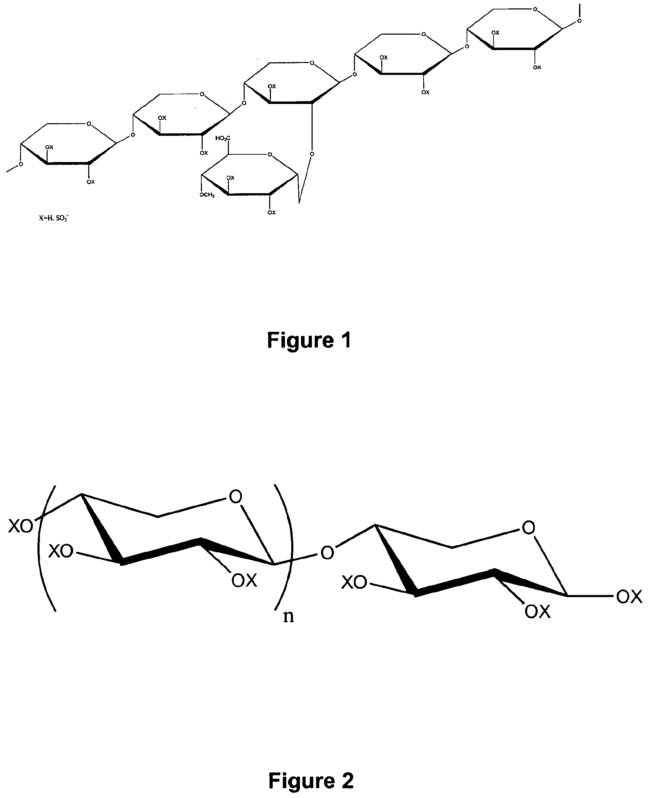

Reference to a "sulfated xylan" includes sulfated xylo-oligosaccharides such as those prepared from the β1-4 xylo-oligosaccharide as well as sulfated xylan pentasaccharides. Sulfated xylans include xylobiose, xylotriose, xylotetraose, xylohexaose, xyloheptaose xylooctaose, xylononaose and xylodecaose (see Figure 2). In a particular embodiment, the sulfated xylan is pentosan polysulfate or PPS. PPS is a heterogenous polysaccharide, prepared by the chemical sulfation of xylan polysaccharide extracted from plants, typically beechwood [Bayol et al, US Patent 4,713,373]. It has been characterized as a sulfated β1-4 linked xylan polymer with a 1-2 linked methylglucuronic acid residue occurring approximately every 10^{th} xylose residue. The molecular weight of PPS is reported to be in the range 1000-6000 Da, with an average molecular weight of ~4500Da ["Elmiron".Pentosan Polysulfate Sodium. Product Monograph.Janssen-Ortho Inc January 2006]. Not withstanding these characteristics, PPS also contains small sulfated xylo- oligosaccharides of degree of polymerization (D.P.) 2-8 *[*Degenhardt et al, Arch Pharm (Weinheim), 334(1):27-9, 2001]. Reference to "sulfated xylan" includes a purified fraction as well as a heterogeneous mixture. The term also includes derivatives and homologs of sulfated xylans.

A method is disclosed for the treatment or prophylaxis of a respiratory condition in a subject, the method comprising administering to the subject an effective amount of a sulfated xylan or a derivative or homolog thereof or a salt thereof for a time and under conditions sufficient for amelioration of symptoms of the respiratory condition.

Although the sodium salts of sulfated carbohydrates are most often used, other cations can be substituted for sodium. Cations substituted for sodium in the formation of salts include, calcium, zinc, ammonium and triethanolamine, but any "pharmacologically acceptable" salt may be used.

In an embodiment, the present disclosure provides a method for the treatment or prophylaxis of a respiratory condition in a subject, the method comprising the administering to the subject an effective amount of a xylan sulfate for a time and under conditions sufficient for amelioration of symptoms of the respiratory condition.

The sulfated xylan is pentosan polysulfate (PPS), as defined by the claims.

Hence, another aspect discloses for a method for the treatment or prophylaxis of a respiratory condition in a subject, the method comprising administering to the subject an effective amount of PPS or a functional derivative or homolog thereof for a time and under conditions sufficient for amelioration of symptoms of the respiratory condition.

Yet another aspect discloses a method for the treatment or prophylaxis of a respiratory condition in a subject, the method comprising administering to the subject an effective amount of PPS or a salt thereof for a time and under conditions sufficient for amelioration of symptoms of the respiratory condition.

The respiratory condition is selected from the list comprising asthma, allergic rhinitis, COPD (including emphysema, chronic bronchitis and obstructive bronchiolitis) and physiologically, biochemically or clinically related conditions.

As with the CSF list above, the list of respiratory conditions means that in one embodiment, it is asthma; in another embodiment it is allergic rhinitis; and in another embodiment it is COPD.

Hence, this aspect is directed to a pharmaceutical composition for treatment or prophylaxis of a respiratory condition selected from the list comprising asthma, allergic rhinitis, COPD (including emphysema, chronic bronchitis and obstructive bronchiolitis).

Another aspect is directed to the treatment or prophylaxis of a respiratory condition selected from the list comprising asthma, allergic rhinitis, COPD (including emphysema) . The pharmaceutical composition comprises PPS.

As indicated above, the condition may also be considered a disease. In one embodiment, the disease or condition is an inflammatory or allergic respiratory or pulmonary condition.

Hence, a method is disclosed for the treatment or prophylaxis of an inflammatory or allergic respiratory, including pulmonary, disease or condition in a subject, said method comprising administering to said subject an effective amount of a xylan sulfate or a derivative or homolog thereof for a time and under condition sufficient to ameliorate symptoms or clinical indications of the disease or condition.

In an example of the present disclosure, the medicament is administered *via* inhalation or as a nasal spray or as nasal drops.

Still another aspect is directed to the use of a sulfated xylan or a derivative or homolog thereof in the manufacture of a medicament for the treatment of a respiratory condition.

The sulfated xylan is a sulfated xylan pentasaccharide, i.e. PPS. Examples of respiratory conditions include pulmonary diseases such as allergic or inflammatory pulmonary diseases including asthma, allergic rhinitis, and COPD (including emphysema, chronic bronchitis and obstructive bronchiolitis).

As indicated above, one particular sulfated xylan is PPS. In another embodiment, the subject is a human. In this regard, another aspect provides a pharmaceutical composition for the treatment or prophylaxis of an inflammatory or allergic respiratory including pulmonary disease or condition in a human.

The sulfated xylans described herein may undergo additional sulfation or other modification. The degree of sulfation, for example, may be modified to modulate binding to protein ligands on cell surfaces. Reference to a "sulfated xylan" or a "PPS" includes their salts.

Another aspect of the present disclosure contemplates a method for producing sulfated xylans or sulfated xylan-like composite molecules that interact with a ligand such as a protein, said method comprising producing a library of sulfated xylans or sulfated xylan-composite molecules and then screening each member of said library or groups of members of said library for any ability to interact with said ligand or to inhibit the interaction between the ligand and sulfated xylan known to interact with the ligand. The library may be different fractions or derivatives of the sulfated xylans. As indicated above, PPS is an example of a sulfated xylan. Examples of ligands include, but are not limited to: a cytokine including an interleukin (e.g. IL-5, IL-4, IL-13, IL-6, IL-7, IL-8, IL-9, IL-10, IL-11, IL-12, IL-3, IL-14, IL-15 or IL-17 family of cytokines including IL-25), interferon (e.g. α-interferon, β-interferon, γ-interferon) or a growth factor including but not limited to G-CSF, M-CSF, GM-CSF, BDNF, CNTF, EGF, EPO, FGF1, FGF2, FGF3, FGF4, FGF5, FGF6, FGF7, FGF8, FGF9, FGF10, FGF11, FGF12, FGF12, FGF13, FGF14, FGF15, FGF16, FGF17, FGF18, FGF19, FGF20, FGF21, FGF22, FGF23, LIF, PDGF, SCF, TGFα, TGFβ, TNFα, TNFβ, TPO, VEGF, GH, NGF, NT 3, NT4, NT5, NT6, NT7, oncostatin M (OSM) and insulin, or a chemokine including but not limited to MCP-1, MCP-2, MCP-3, MCP-4, MCP-5, a member of the MIP-1 family including MIP-1α, MEP-2, eotaxin (eotaxin-1, -2 or -3), PBP (platelet basic protein), SDF-1, PBSF, PF4, RANTES, and the like; an enzyme such as elastase; an enzyme of the cathepsin family, cell adhesion molecules such as PECAM-1; or a soluble or cell- or virus-bound receptor. Particular ligands are IL-5, IL-4, IL-13, G-CSF and M-CSF; particular ligands also include IL-8, MCP-1, MIP-1α eotaxin-1 and eotaxin-2.

There are, of course, any number of assays which may be used to screen for interaction between a sulfated xylan or sulfated xylan-like composite molecule and a ligand or used to screen for inhibition of interaction between a ligand and a sulfated xylan. One assay is a filter binding assay. In this assay, one of a sulfated xylan, or sulfated xylan-like composite molecule, or a ligand is labeled with a reporter molecule capable of providing an identifiable signal such as a fluorescent dye and both molecules are allowed to interact in solution. The resulting mixture is then passed through a filter capable of retarding one of the sulfated xylan or sulfated xylan-like composite molecule or the ligand or only a sulfated xylan-ligand complex or sulfated xylan-like composite molecule-ligand complex.

In one example, the filter is a nitrocellulose filter which retards ligands. In this case, if the sulfated xylan fraction, labeled with a reporter molecule, fails to pass through the filter, then the presence of the reporter signal in the filter indicates binding of the sulfated xylan to the ligand.

Different sulfated xylans will interact with different ligands, or different ligands will interact with different sulfated xylans or both. Accordingly, another assay involves the use of affinity columns carrying immobilized ligands. The sulfated xylans or sulfated xylan-like composite molecules are then passed through the column and the presence of retardation of the sulfated xylans determined. A salt gradient is conveniently used to elute bound sulfated xylans or sulfated xylan-like composite molecules. Once a fraction that binds to a ligand on a column is identified, the fraction can be further analyzed to obtain an indication of the number of different structural entities therein. Such analysis may comprise, for example, anion exchange chromatography, mass spectrometry or electrophoresis.

Once a population of sulfated xylans that bind to a particular ligand has been identified, this fraction itself may be useful as a therapeutic to inhibit interaction between a protein (or other ligand) and a cell surface molecule (such as a HLGAG). The protein (or other ligand) may be cell free or associated with a cell or virus such as a cell surface or viral surface. The sulfated xylan may also be useful as a therapeutic to modulate interaction between a secreted cellular product and extracellular matrix components or between a cell surface protein and extracellular matrix components, or between a protein and its ligand, both or either of which may be cell surface or cell associated. Alternatively, the sulfated xylan may be used as a target to identify natural products or products from a chemical library that mimic the sulfated xylan in terms of binding to a ligand or that inhibits or promotes the interaction between the sulfated xylan and the ligand. These molecules may be antagonists or agonist or chemical analogs of the sulfated xylan. Hence, an "analog" extends to and encompasses any structure which is functionally equivalent in that it binds and/or modulates a ligand in an analogous manner.

Reference herein to "modulate" or "modulation" extends to and encompasses inhibiting and/or promoting an interaction.

Another aspect provides a composition comprising a sulfated xylan and one or more pharmaceutically acceptable carriers, diluents an/or excipients.

In a particular aspect, a composition is disclosed comprising a sulfated xylan which acts as an antagonist to a ligand selected from a cytokine including an interleukin (e.g. IL-5, IL-4, IL-13, IL-6, IL-7, IL-8, IL-9, IL-10, IL-11, IL-12, IL-3, IL-14, IL-15 or IL-17 family of cytokines including IL-25), interferon (e.g. α-interferon, β-interferon, γ-interferon) or a growth factor including but not limited to G-CSF, M-CSF, GM-CSF, BDNF, CNTF, EGF, EPO, FGF1, FGF2, FGF3, FGF4, FGF5, FGF6, FGF7, FGF8, FGF9, FGF10, FGF11, FGF12, FGF12, FGF13, FGF14, FGF15, FGF16, FGF17, FGF18, FGF19, FGF20, FGF21, FGF22, FGF23, LIF, PDGF, SCF, TGFα, TGFβ, TNFα, TNFβ, TPO, VEGF, GH, NGF, NT 3, NT4, NT5, NT6, NT7, oncostatin M (OSM) and insulin, or a chemokine including but not limited to MCP-1, MCP-2, MCP-3, MCP-4, MCP-5, a member of the MIP-1 family including MIP-1α, MIP-2, eotaxin (eotaxin-1, -2 or -3), PBP

(platelet basic protein), SDF-1, PBSF, PF4, RANTES, and the like; an enzyme such as elastase; an enzyme of the cathepsin family, cell adhesion molecules such as PECAM-1; or a soluble or cell- or virus-bound receptor, the composition further comprising one or more pharmaceutical acceptable carriers, diluents and/or excipients.

In a related example the present disclosure is directed to a composition comprising a fraction or derivative of a sulfated xylan which is an antagonist of a ligand selected from the list comprising IL-5, IL-4, IL-13, IL-6, 1L-7, IL-8, IL-9, IL-10, IL-11, IL-12, IL-3, IL-14, IL-15 or IL-17 family of cytokines including IL-25, interferon, G-CSF, M-CSF, GM-CSF, BDNF, CNTF, EGF, EPO, FGF1, FGF2, FGF3, FGF4, FGF5, FGF6, FGF7, FGF8, FGF9, FGF10, FGF11, FGF12, FGF12, FGF13, FGF14, FGF15, FGF16, FGF17, FGF18, FGF19, FGF20, FGF21, FGF22, FGF23, LIF, PDGF, SCF, TGFα, TGFβ, TNFα, TNFβ, TPO, VEGF, GH, NGF, NT 3, NT4, NT5, NT6, NT7, oncostatin M (OSM), insulin, MCP-1, MCP-2, MCP-3, MCP-4, MCP-5, a member of the MIP-1 family including MIP-1α, MIP-2, eotaxin (eotaxin-1, -2 or -3), PBP (platelet basic protein), SDF-1, PBSF, PF4, RANTES, elastase; an enzyme of the cathepsin family, a cell adhesion molecules such as PECAM-1 and a soluble or cell- or virus-bound receptor, said composition further comprising one or more pharmaceutical acceptable carriers, diluents and/or excipients.

Particular molecules which are antagonized include IL-5, IL-4, IL-13, G-CSF and M-CSF; particular molecules also include IL-8, MCP-1, MIP-1α, eotaxin-1 and eotaxin-2.

All such the sulfated xylan referred to above are generically covered by the term "active ingredients". These compositions are proposed for use as pharmaceutical compositions or formulations.

The compositions include sterile aqueous solutions and sterile powders. Such forms are conveniently stable under the conditions of manufacture and storage and are generally preserved against the contaminating action of microorganisms such as bacteria and fungi. The carrier can be a solvent or dilution medium comprising, for example, water, ethanol, polyol (for example, glycerol, propylene glycol and liquid polyethylene glycol, and the like), suitable mixtures thereof and vegetable oils. The proper fluidity can be maintained, for example, by the use of surfactants. The preventions of the action of microorganisms can be brought about by various anti-bacterial and anti-fungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile solutions are prepared by incorporating the active ingredients in the required amount in the appropriate solvent with the active ingredient and optionally other active ingredients as required, followed by sterilization or at least a process to reduce contaminating viruses, bacteria or other biological entities to acceptable levels for administration to a human or animal subject. In the case of sterile powders for the preparation of sterile injectable solutions, suitable methods of preparation include vacuum drying and the freeze-drying technique that yields a powder of active ingredient plus any additionally desired ingredient.

When the active ingredient is suitably protected, it may be orally administered, for example, with an inert diluent or with an assimilable edible carrier, or it may be enclosed in hard or soft shell gelatin capsule, or it may be compressed into tablets. For oral therapeutic administration, the active ingredient may be incorporated with excipients and used in the form of ingestible tablets, buccal tablets, troches, capsules, elixirs, suspensions, syrups, wafers and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions is such that a suitable dosage will be obtained. Particular compositions or preparations are prepared so that an oral dosage unit form contains between about 0.1 µg and 200 mg of active compound. Alternative dosage amounts include from about 1 µg to about 1000 mg and from about 10 µg to about 500 mg. These dosages may be per individual or per kg body weight. Administration may be per second, minute, hour, day, week, month or year.

The tablets, troches, pills and capsules and the like may also contain the components as listed hereafter. A binder such as gum, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added. When the dosage unit form is a capsule, it may contain, in addition to materials of the above type, a liquid carrier. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

Pharmaceutically acceptable carriers and/or diluents include any and all solvents, dispersion media, coatings, anti-bacterial and anti-fungal agents, isotonic and absorption delaying agents and the like. The use of such media and agents for pharmaceutical active substances is well known in the art and except insofar as any conventional media or agent is incompatible with the active ingredient; their use in the therapeutic compositions is contemplated. Supplementary active ingredients can also be incorporated into the compositions.

The composition may also be formulated for local or topical administration. Techniques for formulation and administration may be found in "Remington's Pharmaceutical Sciences", Mack Publishing Co., Easton PA., 16th edition, 1980, Ed. By Arthur Osol. Thus, for local or topical administration, the subject compositions may be formulated in any suitable manner, including, but not limited to, creams, gels, oils, ointments, solutions, suspensions, powders, mists or aerosols. For transmucosal administration, penetrants appropriate to the barrier to be permeated are used in the formulation. Such penetrants are generally known in the art and include, but are not restricted to, benzalkonium chloride, digitonin, dihydrocytochalasin B, and capric acid.

The compositions described herein, in the form of lotions, creams or gels may contain acceptable diluents or carriers to impart the desired texture, consistency, viscosity and appearance. Acceptable diluents and carriers are familiar to those skilled in the art and include, but are not restricted to, ethoxylated and nonethoxylated surfactants, fatty alcohols, fatty acids, hydrocarbon oils (such as palm oil, coconut oil, and mineral oil), cocoa butter waxes, silicon oils, buffering agents, cellulose derivatives, emulsifying agents such as non-ionic organic and inorganic bases, preserving agents, wax esters, steroid alcohols, triglyceride esters, phospholipids such as lecithin and cephalin, polyhydric alcohol esters, fatty alcohol esters, hydrophilic lanolin derivatives, and hydrophilic beeswax derivatives.

The present invention is further described by the following non-limiting Examples.

In the Examples, the agents, reagents and methods referred to below are employed:

### Pentosan Polysulfate (PPS)

Pentosan polysulfate (PPS) is available under various brand names such as: Elmiron; Elmiron; Fibrase; Fibrezym; Hémoclar; Pentosanpolysulfat SP 54; Polyanion; SP54; Tavan-SP; Thrombocid; Cartorphen Vet; and Pentosan equine. One manufacturer of the active ingredient is Bene PharmaChem. The chemistry registry numbers for PPS include 37300-21-3, 9014-63-5, 11096-31-4, 39432-58-1, 42613-02-5 and 104783-23-5. PPS is used as an example of a sulfated xylan.

### Specialized reagents

### (1) IL-5 and IL-4

Recombinant human IL-4 and IL-5 are prepared using a baculovirus expression system. Recombinant human IL-4 is also expressed in an *E. coli* expression system. Recombinant human IL-4 or IL-5 carrying site directed mutations are expressed using a baculovirus expression system. The resulting proteins are then purified by affinity chromatography on HiTrap activated NHS columns derivatized with hydrazine and coupled with either the monoclonal antibody H30, or the monoclonal antibody TRFK-5, both of which recognize IL-5, or the monoclonal antibody, 11B4, which recognizes IL-4.

### MALDI-MS

Matrix assisted laser desorption ionization mass spectrometry (MALDI MS) was performed using the procedure described by Venkataraman et al, Science 286(5439):537-542, 1999. the basic peptide (RG)₁₉R was prepared as the trifluoroacetate salt by Auspep (Melbourne, Australia). Approximately 20µg of AG-1 X2 anion exchange resin in the hydroxide form (Bioard, Sydney, Australia) was added to an ice-cold aliquot (100µL) of 50µM peptide. The resulting suspension was centrifuged briefly and maintained in an icebath. An aliquot of peptide (1µL) was mixed with 10mg/mL caffeic acid in 50% v/v acetonitrile (8µL) and 5-100µM sample (1µL), 1µL spotted onto a stainless steel sample plate and allowed to dry. MALDI MS spectra were acquired in the linear mode by using a PerSeptive Biosystems (Applied Biosystems, Melbourne, Australia) Voager reflectron time-of-flight instrument fitted with a 337-nm nitrogen laser. Delayed extraction was used to increase resolution (22kV, grid at 93%, guide wire at 0.15%, pulse delay 150ns, low mass gate at 2000, 50 shots averaged). Alternatively, spectra were acquired with an ABI Model 4800 MALDI instrument. Mass calibration was achieved by external calibration with the peptide calibration mixture provided by the manufacturer. The mass of the oligosaccharide was deduced by subtracting the mass of the (RG)₁₉R peptide observed for that sample.

### Sulfated xylo-oligosaccharides

Xylo-oligosaccharide mixture (5g) was dissolved in 100ml DMF. Pyridine-sulfur trioxide complex (36g) was added and the solution stirred for 20 hours. The reaction was quenched by addition of water (500ml) and adjusted to pH 6 with tributylamine. This solution was dialyzed in 2 x 10L of water using 3.5kDa MWCO dialysis tubing. The dialysate was concentrated by ultrafiltration using a 1kDa MWCO membrane and was fractionated by preparative reversed-phase ion-pairing HPLC by repeated injection.

Example conditions:
- Column:: Phenomenex Axia 100 x 21.2mm Luna C18 (2) fitted with a security guard cartridge (or suitable substitute).
- Eluent A:: 8mM tributylamine in 20% v/v acetonitrile adjusted to pH 5.8 with acetic acid.
- Eluent B:: 80% v/v acetonitrile.
- Flow:: 20ml/min.
- Detector:: Evaporative light scattering with split flow of ~1:40 achieved with a T-piece.

The size of the fractions were assessed by size-exclusion chromatography using two of 250 x 7.8mm BioSep 2000 columns (Phenomenex, or equivalent, e.g. TSK2000W) connected in series and eluted with 0.25 M NH₄Cl at 0.5ml/min. The eluent was monitored with a refractive index detector. The appropriate size fraction(s) were concentrated in a rotary evaporator and the resulting solution lyophilized to remove excess tributylamine acetate. The tributylamine salts can be used directly, or alternatively converted to the sodium salts by the following procedure. The residue was dissolved in water and the resulting solution adjusted to pH 5.5 with acetic acid. This solution was applied to a 4 x 2cm column of Dowex 50WX8 (NA⁺ form) 2 x 4cm and washed with three column volumes of water. The flow through and washings were collected and pooled. The pooled washings were adjusted to pH 7 and dialyzed by repeated ultrafiltration with a 1kDa MWCO membrane. The concentrated solution was lyophilized to yield a white powder.

### Sulfated malto-oligosaccharides

Using purified oligosaccharides as starting materials these can be prepared by the procedures described in either US Patent Nos. 4,021,544; US 6,271,215 and US 5,541,166. these materials can also be prepared by a procedure analogous to that described above, whether starting from a purified oligosaccharide or a mixture of oligosaccharides. For example, maltohexaose (0.1g, Sigma-Aldrich) was dissolved in DMF (10ml) and pyridine.sulfur trioxide complex (1g) added. The mixture was stirred at room temperature for 20 hours and the product allowed to separate as an oil. The supernatant was decanted and the residue dissolved in water (15ml) and adjusted to pH 6 with tributylamine. The crude material was purified by HPLC and further processed as described above for the sulfated xylo-oligosaccharides. Sulfated maltopheptaose was prepared in a similar manner.

Mixtures of oligosaccharides can also be sulfated and then fractionated as described for the sulfated xylo-oligosaccharides. For example, pentrup syrup from Hayashibara International (5g) was dissolved in 100ml DMF. Pyridine-sulfur trioxide complex (54g) was added and the mixture was stirred at room temperature for 20 hours and the product allowed to separate as an oil. The supernatant was decanted and the residue dissolved in water (15ml) and adjusted to pH 6 with tributylamine. The mixture was fractionated by HPLC and further processed as described above for the sulfated xylo-oligosaccharides.

### EXAMPLE 1

### BIAcore Screening assay

The optical phenomenon of surface plasmon resonance is used to monitor physical interactions between molecules. Passing a solution of a potential protein ligand (e.g. IL-4, IL-5 or eotaxin) over a sensor surface to which a target (e.g. heparin) is coupled monitors the real-time binding of protein ligands to the immobilized target. Detection is achieved by measuring refractive index changes very close to the sensor surface. When the refractive index is altered, the angle at which plasmon resonance occurs changes and this change directly correlates with the amount of protein interacting with the surface. A BIAcore 2000 is conveniently used. It is very sensitive and its microfluidics ensures that only small amounts of material are required.

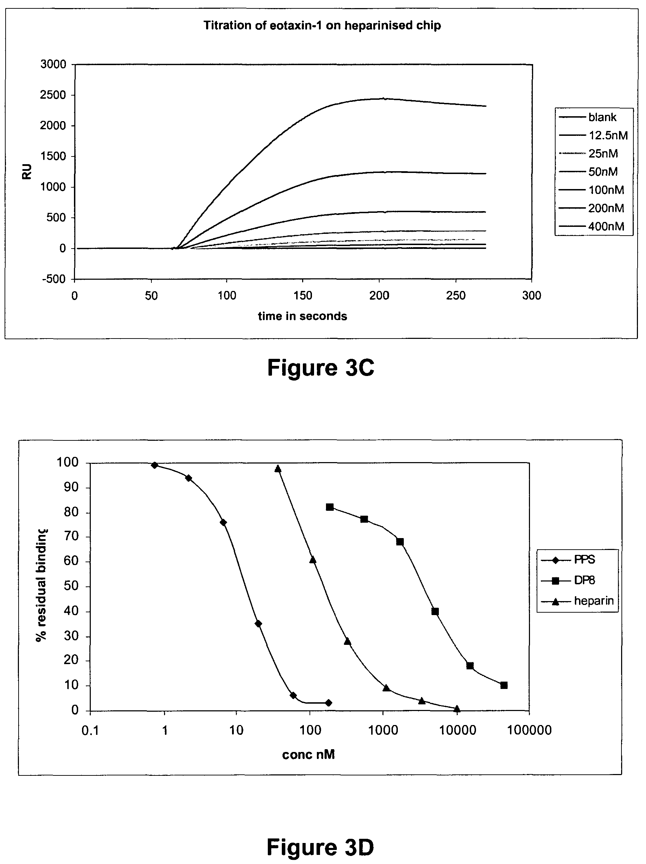

Biotinylated heparin is immobilized on the biosensor chip. Biotinylation occurs *via* amino groups, or reducing termini modified with ammonia by reductive amination, using sulfo-NHS-biotin. Solutions containing potential protein ligands of interest are injected over the sensor chip surface, and the binding is measured in real time (Fernig, In: Proteoglycan protocols, Ed. R.V. Iozzo, Humana Press, Totowa, NJ, USA, 2001). Baculovirus expressed recombinant human IL-4 (rhIL-4) and baculovirus expressed recombinant human IL-5 (rhIL-5) readily bind to heparin immobilized by this method (see PCT/AU2005/000551). Binding is specific, as there is little interaction with sensor chips lacking heparin with IL-4 or IL-5, and binding is inhibited by exogenous heparin. Similarly, recombinant human eotaxin (CCL11) expressed in *E. coli* binds readily to immobilized heparin and binding is specific as there is little eotaxin binding to sensor chips lacking heparin. Moreover, the binding is concentration dependent (Figure 3C). The binding of eotaxin to immobilized heparin is inhibited by exogenous heparin with an IC₅₀ of approximately 160nM.

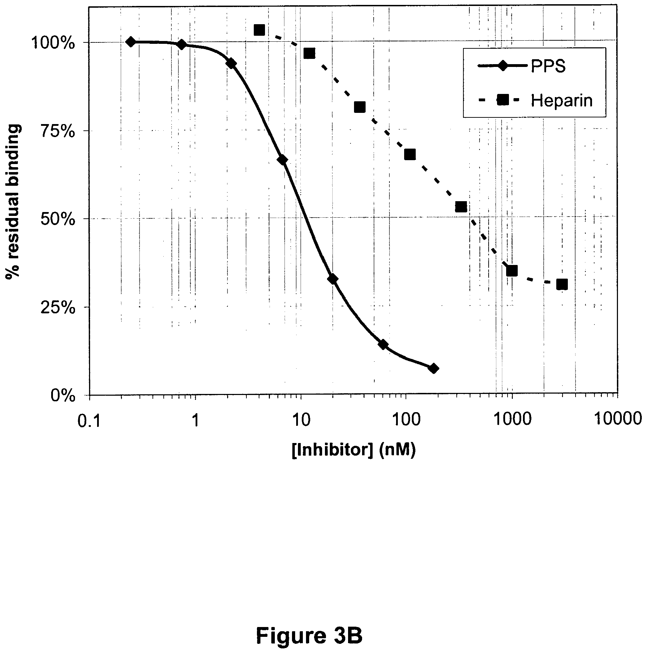

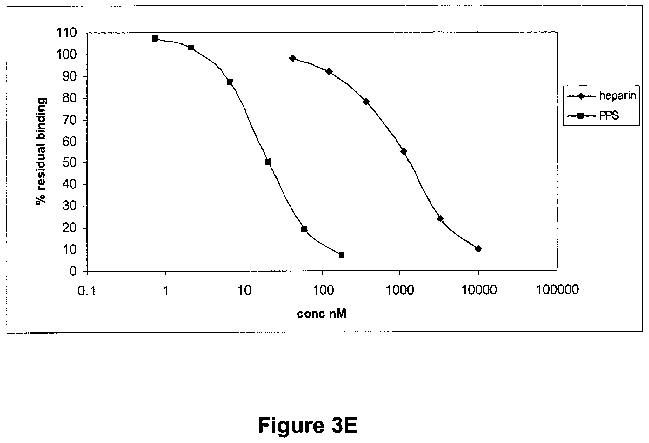

Preparations of PPS inhibit the binding of IL-4, IL-5 and eotaxin to heparin immobilized on the BIAcore chip. Titration experiments indicate that PPS is a more potent inhibitor of IL-4 binding to immobilized heparin than heparin itself with an IC₅₀ of around 25-80nM versus the IC₅₀ of 3.3-1µM which is obtained with heparin (Figure 3A). Similar titration experiments indicate that PPS is also a more potent inhibitor of IL-5 binding to immobilized heparin than heparin itself with an IC₅₀ of around 4-10nM versus the IC₅₀ of around 30-100nM which is obtained with heparin (Figure 3B). PPS is also a more potent inhibitor of eotaxin-1 and eotaxin-2 binding to immobilized heparin than heparin itself with an IC₅₀ of around 15nM (Figure 3D and 3E). The size of the sulfated xylose polysaccharides is an important component of its ability to bind to eotaxin and thereby block eotaxin binding to heparin. A sulfated xylan of D.P. 8 was less able to inhibit eotaxin-1 binding to immobilized heparin having an IC₅₀ of approximately 3.5µM (Figure 3D).

These data indicate that PPS binds to IL-4 at the site where heparin binds and that its binding to this region is more stable than that of heparin binding. These data similarly indicate that PPS binds to IL-5 at the site where heparin binds and that it's binding to this region is more stable than that of heparin binding. The eotaxin data also indicate that PPS binds to eotaxin-1 and eotaxin-2 at a site in the vicinity of where heparin binds and that it's binding to this region is more stable than that of heparin binding.

### EXAMPLE 2

### Functional analyses of pentosan polysulfate on the asthma and allergic rhinitis protein target, IL-5

PPS inhibited the proliferation of an IL-5 responsive cell line. This occurs at very low doses and is not due to a toxic effect of the PPS as other similarly sulfated polysaccharides have no affect in this assay. These experiments are performed with the IL-5 responsive cells, Ba/F-IL-5. The Ba/F-IL-5 cells were derived from the Ba/F3 cell line.

The Ba/F3 cell line was transformed to be both IL-5 dependent and to express luciferase by co-transfection of the cells with pGL3 control vector (Promega, USA) and pEE6hcmv-IL-5Rα. The control vector, pGL3 expresses a modified luciferase under the direct control of the SV40 promoter and enhancer, but contains no selectable marker. To prepare pEE6hcmv-hIL-5Rα a full length human IL-5 receptor α chain (hIL-5R-α) was cloned by RT PCR from HL60 cells. The preparation of the Ba/F-IL-5 cells has been described by Coombe et al, Journal of Immunological Methods 215: 145-150, 1998. The Ba/F-IL-5 cells may be further modified by co-transfection with pPGK-puromycin-luciferase, a vector containing luciferase under the control of the SV40 promotor with the selectable marker puromycin.

After transfection, positive transfectants are selected in 3 µg/ml puromycin. The positive transfectants are then cloned to produce a line with detectable luciferase expression. The proliferation assays are carried out in 96-well microplates suitable for such assays (Falcon). The wells are flat bottomed, with white sides and a clear bottom. Cells are washed to remove any cytokine in the growth medium and then resuspended in RPMI/5% w/v FCS. The cells are counted with a Coulter Z2 Particle Counter and Size Analyzer (Coulter Electronics, England) and routinely 1.6 x 10⁴ cells are added to microplate wells that contain either no IL-5 (negative control) or various dilutions of IL-5. When the effect of PPS, or other sulfated polysaccharides is to be measured, the wells also contain various concentrations of these molecules.

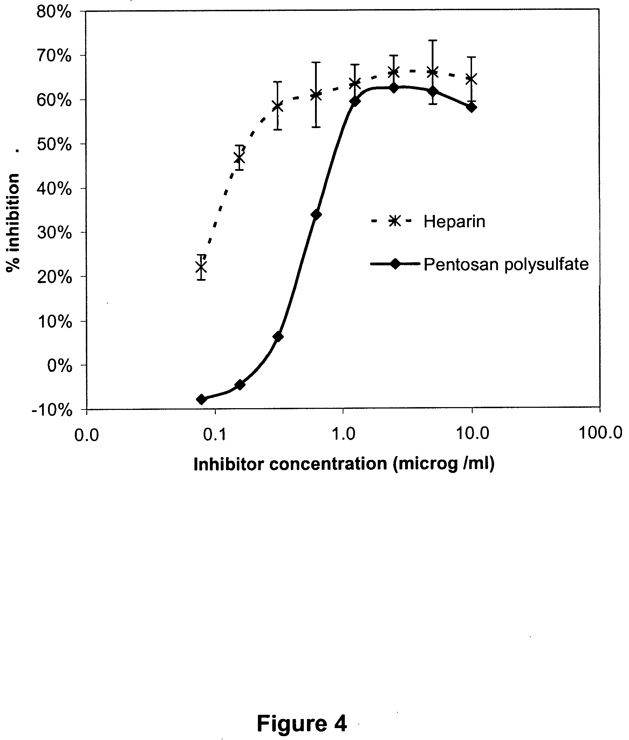

The cells proliferate for 24 hours at 37°C in a humidified atmosphere, after which the luciferase activity is measured by the addition of 50 µl of luciferase substrate buffer (50 mM Tris-HCl, pH 7.8, 15 mM MgSO₄, 33.3 mM DTT, 0.1 mM EDTA, 0.5 mM Na-luciferin, 0.5 mM ATP, 0.25 mM lithium Co A and 0.5% v/v Triton X-100). Immediately after the addition of the luciferase buffer the plate is assayed for luciferase activity. Light emissions are detected on a Victor 1420 Multi-label counter (Wallac, Turku, Finland). Using this assay it has been demonstrated that PPS is an effective inhibitor of IL-5 dependent Ba/F-IL-5 cell proliferation (Figure 4).

The ability of PPS to block the binding of fluorescent-labeled IL-5 (IL-5-Alexa 488) to its receptor is also tested. The IL-5 responsive cells TF-1.8 and Ba/F-IL-5 are used for these experiments. The TF-1.8 cells are a subclone of the TF-1 cells that have been selected for growth in IL-4 or IL-5. TF-1 cells were originally established from a bone marrow sample from a male with severe pancytopenia. These cells are dependent on IL-3 or GM-CSF for long term growth and are responsive to a variety of cytokines including IL-4, but not IL-5. The binding of fluorescent-labeled IL-5 to TF-1.8 cells or Ba/F-IL-5 in the presence or absence of PPS is measured by flow cytometry.

To fully appreciate the effect of the PPS on IL-5 activity, primary cells responsive to IL-5 are examined. Human peripheral blood eosinophils are isolated from healthy donors by a CD16 negative selection protocol. A common way to assess eosinophil activation is by monitoring their adhesion to immobilized IgG. The number of eosinophils bound to immoblized IgG when IL-5 is presented with the selected oligosaccharides, or with heparin or heparan sulfate, compared to IL-5 alone, is determined by measuring myeloperoxidase activity. Eosinophils separated from peripheral blood do not survive more than four days in the absence of cytokine. The effect of IL-5 in the presence of PPS on eosinophil survival relative to that of IL-5 alone is assessed at a range of cytokine concentrations. Eosinophil survival is determined by flow cytometry following propidium iodide staining.

### EXAMPLE 3

### Functional analyses of pentosan polysulfate on the asthma target protein, IL-4

PPS inhibited the proliferation of a human IL-4 responsive cell line. This occurs at very low doses and is not due to a toxic effect of the pentosan polysulfate because other, similarly sulfated polysaccharides, at the same concentrations of IL-4 and polysaccharide have no effect. These experiments utilize the TF-1.8 cells. TF-1.8 cells are a subclone of the TF-1 cells that have been selected for growth in IL-4 or IL-5. TF-1 cells were originally established from a bone marrow sample from a male with severe pancytopenia. These cells are dependent on IL-3 or GM-CSF for long term growth and are responsive to a variety of cytokines including IL-4, but not IL-5.

TF-1.8 cells have been transfected with the firefly luciferase gene contained in the expression vector, pPGK-puromycin-luciferase (Coombe *et al,* 1998, *supra*). The positive transfectants are cloned to produce a line with good luciferase expression. The proliferation assays are carried out in 96-well microplates suitable for such assays (Falcon). The wells are flat bottomed, with white sides and a clear bottom. Cells are washed to remove any cytokine in the growth medium and then resuspended in RPMI/5% w/v FCS. The cells are counted with a Coulter Z2 Particle Counter and Size Analyzer (Coulter Electronics, England) and routinely 2.5 x 10⁴ cells are added to microplate wells that contain either no IL-4 (negative control) or various dilutions of IL-4. When the effect of PPS, or other sulfated polysaccharides is to be measured, the wells also contain various concentrations of these molecules.

The cells proliferate for 48 hours at 37°C in a humidified atmosphere, after which the luciferase activity is measured by the addition of 50 µl of luciferase substrate buffer (50 mM Tris-HCl, pH 7.8, 15 mM MgSO₄, 33.3 mM DTT, 0.1 mM EDTA, 0.5 mM Na-luciferin, 0.5 mM ATP, 0.25 mM lithium Co A and 0.5% v/v Triton X-100). Immediately after the addition of the luciferase buffer the plate is assayed for luciferase activity. Light emissions are detected on a Victor 1420 Multi-label counter (Wallac, Turku, Finland).

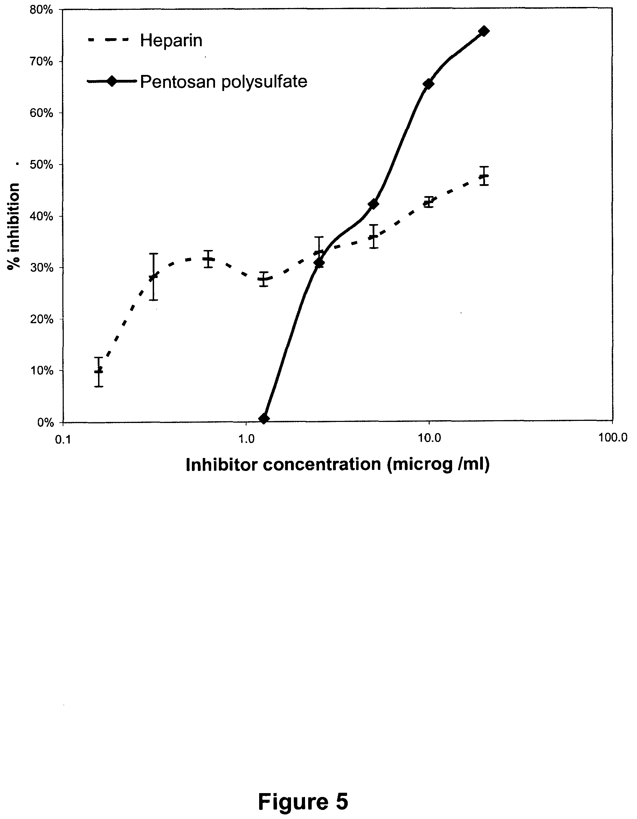

Using this assay, the inventors demonstrated that PPS markedly inhibits the IL-4 dependent proliferation of TF-1.8 cells (Figure 5).

The ability of fluorescent-labeled IL-4 to bind to its receptor, in the presence or absence of pentosan polysulfate, is examined. These experiments are performed using different concentrations of IL-4, which has been conjugated with AlexaFluor-488, and PPS. Comparisons with other sulfated polysaccharides shown not to have activity in inhibiting the IL-4 dependent proliferation of TF-1.8 cells, will demonstrate specificity.

### EXAMPLE 4

### Pentosan polysulfate does not inhibit the activity of GM CSF or IL-2

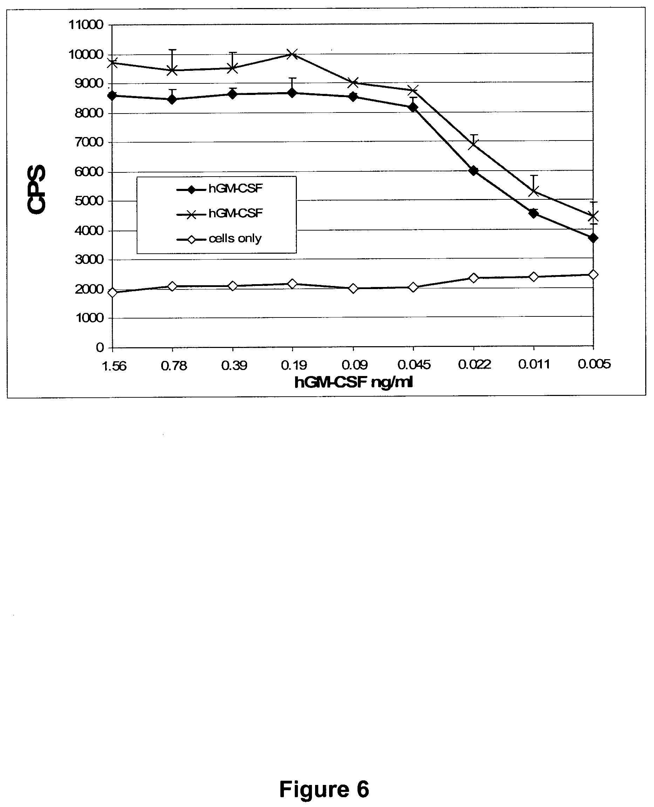

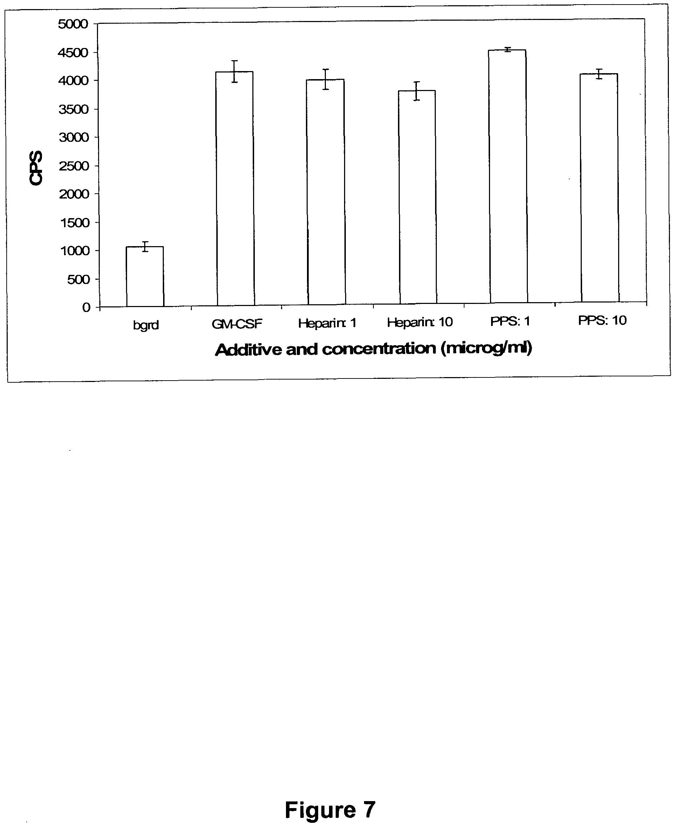

TF-1.8 cells were derived from TF-1 cells that respond to human GM-CSF (granulocyte-macrophage colony stimulating factor). TF-1.8 cells retained their responsiveness to GM-CSF. Routinely 2.5 x 10⁴ cells are added to microplate wells that contain either no GM-CSF (negative control) or various dilutions of GM-CSF. The cells are cultured for 48 hours at 37°C in a humidified atmosphere, after which the luciferase activity is measured by the addition of 50 µl of luciferase substrate buffer (50 mM Tris-HCl, pH 7.8, 15 mM MgSO₄, 33.3 mM DTT, 0.1 mM EDTA, 0.5 mM Na-luciferin, 0.5 mM ATP, 0.25 mM lithium Co A and 0.5% v/v Triton X-100). Immediately after the addition of the luciferase buffer the plate is assayed for luciferase activity. Light emissions are detected on a Victor 1420 Multi-label counter (Wallac, Turku, Finland). A titration of GM-CSF established that these cells are dependent upon this growth factor (Figure 6). When the effect of PPS, or other sulfated polysaccharides is to be measured, the wells also contain various concentrations of these molecules as well as the GM-CSF. These experiments have indicated that concentrations of 10µg/ml and 1 µg/ml of PPS reproducibly have no effect on the TF-1.8 cell proliferation obtained with 0.025ng/ml of GM-CSF (Figure 7).

The murine cytotoxic T lymphocytic line (CTLL) is a subclone of T cells derived from a C57b1/6 mouse. The cells require interleukin-2 (IL-2) for growth and are used to assay for its presence in conditioned media. The cells are responsive to both murine and human IL-2. CTLL cells have been transfected with the firefly luciferase gene contained in the expression vector, pPGK-puromycin-luciferase (Coombe *et al,* 1998, *supra*). The positive transfectants are cloned to produce a line with good luciferase expression and these cells are called CTL-Luc. The proliferation assays are carried out in 96-well microplates suitable for such assays (Falcon). The wells are flat bottomed, with white sides and a clear bottom. Cells are washed to remove any cytokine in the growth medium and then resuspended in RPMI/5% w/v FCS. The cells are counted with a Coulter Z2 Particle Counter and Size Analyzer (Coulter Electronics, England) and routinely 1.6 x 10⁴ cells are added to microplate wells that contain either no recombinant human IL-2 (rhIL-2) (negative control) or various dilutions of rhIL-2. When the effect of PPS, or other sulfated polysaccharides is to be measured, the wells also contain various concentrations of these molecules.

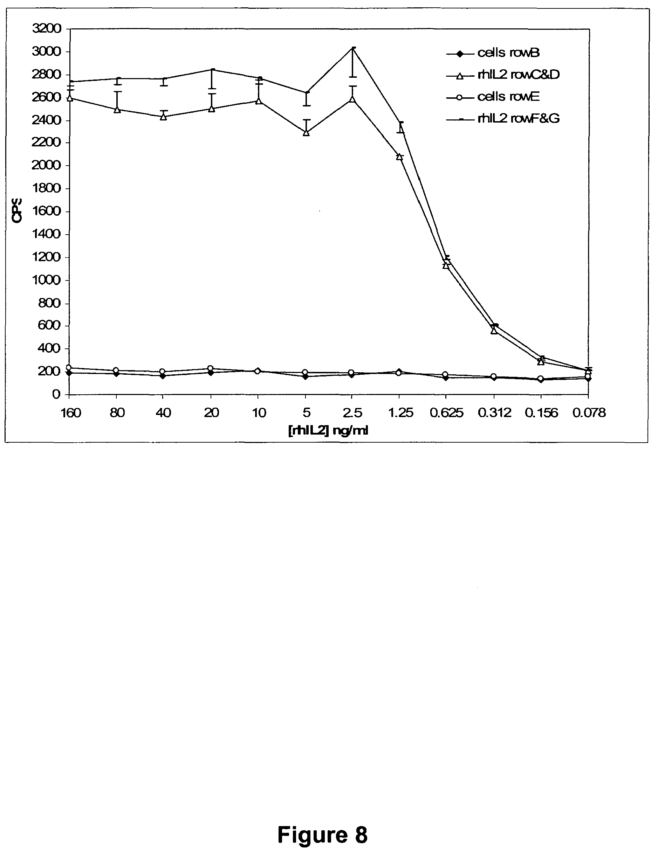

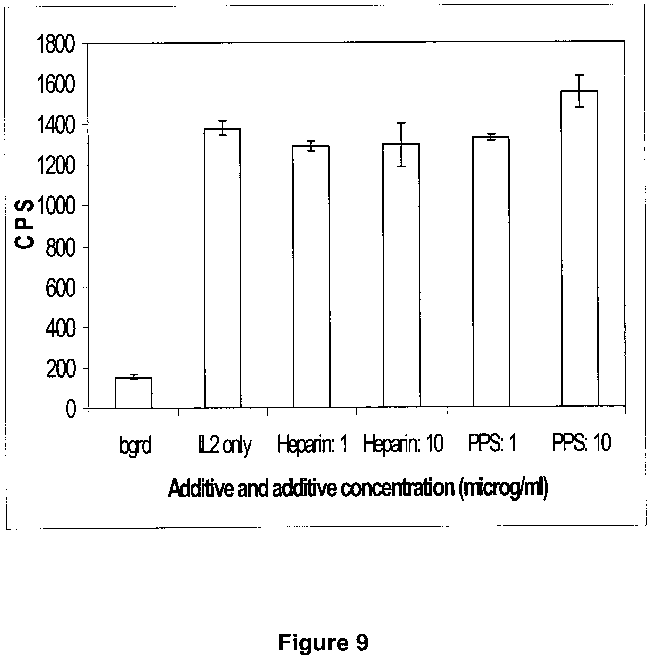

The CTL-Luc cells proliferate for 24 hours at 37°C in a humidified atmosphere, after which the luciferase activity is measured by the addition of 50 µl of luciferase substrate buffer (50 mM Tris-HCl, pH 7.8, 15 mM MgSO₄, 33.3 mM DTT, 0.1 mM EDTA, 0.5 mM Na-luciferin, 0.5 mM ATP, 0.25 mM lithium Co A and 0.5% v/v Triton X-100). Immediately after the addition of the luciferase buffer the plate is assayed for luciferase activity. Light emissions are detected on a Victor 1420 Multi-label counter (Wallac, Turku, Finland). Experiments in which the concentration of rhIL-2 is titrated indicate that CTL-Lucs are dependent upon IL-2 for proliferation (Figure 8). Experiments in which the CTL-Luc cells are cultured in the presence of either 10µg/ml or 1µg/ml of PPS reproducibly have no effect on the proliferation of CTL-Luc cells obtained with 1.25ng/ml of rhIL-2. (Figure 9). These experiments with the IL-2 and GM-CSF responsive cell lines indicate that pentosan polysulfate does not interact with these cytokines in a manner that affects their proliferative activity. These experiments also indicate that PPS is not toxic to cytokine dependent lymphocytic cell lines.

### EXAMPLE 5

### Sulfated xylans display a size dependent increase in anti-IL-5 and anti-IL-4 activity

A number of different sized sulfated xylan preparations have been prepared and it is shown that the size of the saccharide is an important component of its anti-IL-5 activity. These experiments utilize the Ba/F-IL-5 cells transfected with luciferase. Routinely, 1.6 x 10⁴ cells are added to microplate wells that contain either no IL-5 (negative control) or IL-5 (0.4ng/ml) or IL-5 (0.4ng/ml) and various dilutions of pentosan polysulfate, or one of the different sulfate xylan preparations that possess different numbers of saccharide units. The cells are allowed to proliferate for 24 hours at 37°C in a humidified atmosphere, after which the luciferase activity is measured by the addition of 50 µl of luciferase substrate buffer (50 mM Tris-HCl, pH 7.8, 15 mM MgSO₄, 33.3 mM DTT, 0.1 mM EDTA, 0.5 mM Na-luciferin, 0.5 mM ATP, 0.25 mM lithium Co A and 0.5% v/v Triton X-100). Immediately after the addition of the luciferase buffer the plate is assayed for luciferase activity. Light emissions are detected on a Victor 1420 Multi-label counter (Wallac, Turku, Finland). Using this assay it has been demonstrated that although pentosan polysulfate is an effective inhibitor of IL-5 dependent Ba/F-IL-5 cell proliferation small polysaccharides comprising 2, 3 or 4 sulfated xylose units 1-4 linked were not effective inhibitors when used at 1µg/ml (Table 2). Larger polysaccharides comprising 5, 6 or 7 sulfated xylose units linked 1-4 inhibited IL-5 dependent Ba/F-IL-5 cell by approximately 15-16%, whereas the sulfated xylan of D.P. 8 inhibited by approximately 24% and a mixture of sulfated xylose polysaccharides all larger than an octasaccharide inhibited by 40%. PPS was a potent inhibitor at 1µg/ml giving an inhibition of around 55% in this assay.

The size of the sulfated xylose polysaccharides is also an important component of its anti-IL-4 activity. These experiments utilise the TF-1.8 cells transfected with luciferase. Routinely, 2.5 x 10⁴ cells are added to microplate wells that contain either no IL-4 (negative control) or IL-4 (2.5ng/ml) or IL-4 (2.5ng/ml) and various dilutions of PPS, or one of the different sulfate xylan preparations that possess different numbers of saccharide units. The cells are allowed to proliferate for 48 hours at 37°C in a humidified atmosphere, after which the luciferase activity is measured by the addition of 50 µl of luciferase substrate buffer (50 mM Tris-HCl, pH 7.8, 15 mM MgSO₄, 33.3 mM DTT, 0.1 mM EDTA, 0.5 mM Na-luciferin, 0.5 mM ATP, 0.25 mM lithium Co A and 0.5% v/v Triton X-100). Immediately after the addition of the luciferase buffer the plate is assayed for luciferase activity. Light emissions are detected on a Victor 1420 Multi-label counter (Wallac, Turku, Finland). Using this assay it has been demonstrated that although PPS is an effective inhibitor of IL-4 dependent TF-1.8 cell proliferation smaller polysaccharides comprising 2, 3 or 4 sulfated xylose units 1-4 linked were totally ineffective inhibitors when used at either 2.5µg/ml or 5µg/ml (Table 3). The larger polysaccharides comprising 5, 6, or 7 sulfated xylose units linked 1-4 inhibited IL-4 dependent TF-1.8 cell by approximately 15-29% when used at 2.5µg/ml, whereas the octasaccharide and a mixture of sulfated xylose polysaccharides all larger than an octasaccharide and used at 2.5µg/ml inhibited by approximately 21%. PPS was a potent inhibitor at 2.5µg/ml giving an inhibition of around 48% in this assay.

**Table 2**

| **Inhibition of IL-5 induced BA/F cell proliferation in the presence of sulfated xylooligosaccharides of the specified D.P. and PPS. The carbohydrates were present at 1µg/ml. and IL-5 was added at 0.39 ng/ml..** | |
|---|---|
| | Percentage Inhibition |
| D.P. 2 | 17% |
| D.P. 3 | 13% |
| D.P. 4 | 11% |
| D.P. 5 | 17% |
| D.P. 6 | 16% |
| D.P. 7 | 15% |
| D.P. 8 | 24% |
| PPS | 55% |

**Table 3**

| **Inhibition of IL-4 induced TF-1.8 cell proliferation in the presence of sulfated xylooligosaccharides of the specified D.P. and PPS. IL-4 was added at 2.5 ng/ml.** | | |
|---|---|---|
| | Percentage Inhibition | |
| Concentration of Inhibitor | 2.5 µg/ml | 5.0 µg/ml |
| D.P. 2 | -21% | -18% |
| D.P. 3 | -16% | -16% |
| D.P. 4 | -5% | 4% |
| D.P. 5 | 15% | 22% |
| D.P. 6 | 29% | 35% |
| D.P. 7 | 26% | 38% |
| D.P. 8 | 21% | 37% |
| PPS | 48% | 58% |

### EXAMPLE 6

### Pentosan polysulfate inhibits elastase activity

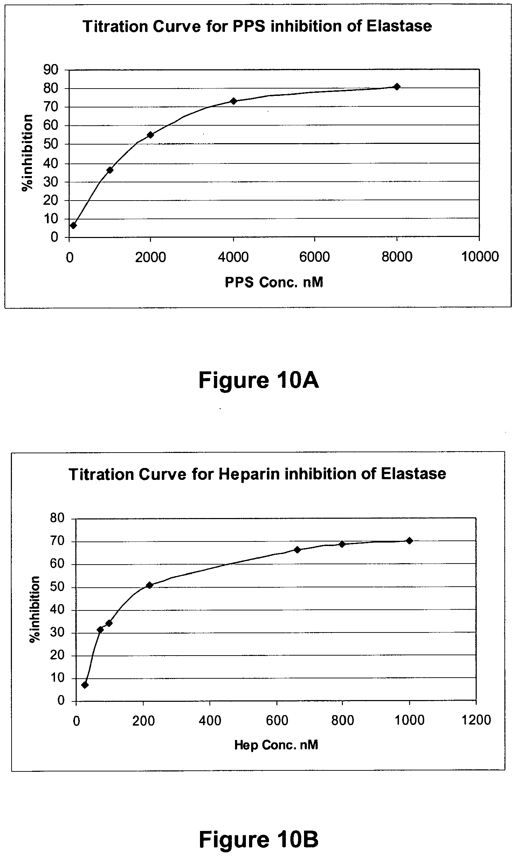

Elastase is a protein that is a potential target for the treatment of COPD. Elastase assays were performed in 96-well plastic microplates for easy quantification by the fluorescent plate reader. Human leukocyte elastase (5nM/well) was incubated in the presence of absence of pentosan polysulfate, or other sulfated polysaccharides, with the fluorogenic substrate MeOSuc-Ala-Ala-Pro-Val-amido-methylcoumarin (20µM/well) in a sodium phosphate buffer, pH 7.4. The mixture was incubated at 37° C for 60 minutes before the reaction was stopped by the addition of 10µl/well of 250 nM acetic acid and the mixture transferred to a 96-well microplate with fluorescence being measured using an excitation wavelength of 355nm and an emission wavelength of 460nm. Various concentrations of inhibitors were used to allow the calculation of the concentration of polysaccharide required to inhibit enzyme activity by 50% (IC₅₀). These data indicated that PPS inhibited elastase activity having an IC₅₀ in this assay of approximately 1.8µM, which compares to the heparin IC₅₀ of approximately 200nM (Figure 10A and B).

Elastase activity was also measured using a physiological substrate, collagen. For these experiments collagen was coated onto the wells of a microplate. Tris buffer (50 µl) was added together with 25 µl of either inhibitor (PPS or other sulfated polysaccharides) or buffer. The plate was incubated at 37 ° C for 30 minutes, before staining the wells for protein. After drying the wells at room temperature the remaining colour was dissolved by adding 200 µl of lysis solution (10% w/v SDS in DMSO) and the OD measured at 590 mm. Control experiments include collagen without enzyme digestion as 100% values and with enzyme digestion but without inhibitor as 0% values. In these experiments PPS was an effective elastase inhibitor.

### EXAMPLE 7

### Pentosan polysulfate inhibits leukocyte infiltration in an allergic rhinitis animal model

An allergic rhinitis model in the guinea pig was used. The guinea pigs are sensitized to ovalbumin (OVA) twice (on days 0 and 7) by an intraperitoneal injection of 0.5ml saline containing 100mg Al(OH)₃ and 2µg OVA. Three weeks after the last sensitization, animals are anaesthetized and the exposure of the nasal cavity to allergen is performed by dropping OVA solution at 20mg/ml into bilateral nasal cavities. For the negative control the animals receive sensitization and challenge with saline. The animals are pretreated with either vehicle or drug (pentosan polysulfate or Budesonide) 30 min prior to intranasal instillation of OVA. Vehicle or drugs are administered, 25µl - 50µl/nostril. A comparison of the pentosan polysulfate with Budesonide, as the reference compound, is included. Animals are terminated and all parameters measured eight hours after the provocation.

The nasal mucosal barrier permeability is assessed by measuring the leakage of protein-rich and non-sieved plasma into the nasal cavities. The amount of extravasated plasma is indicated as nasal lavage levels of total protein or albumin. Nasal lavage fluid is collected by gently rinsing the nasal cavities with phosphate buffered saline. The cells in this fluid are centrifuged and resuspended in phosphate buffered saline and counted using a semiautomated haematology analyzer. The cell composition of the nasal lavage is determined after a cytospin and staining with May Grynwald Giemsa.

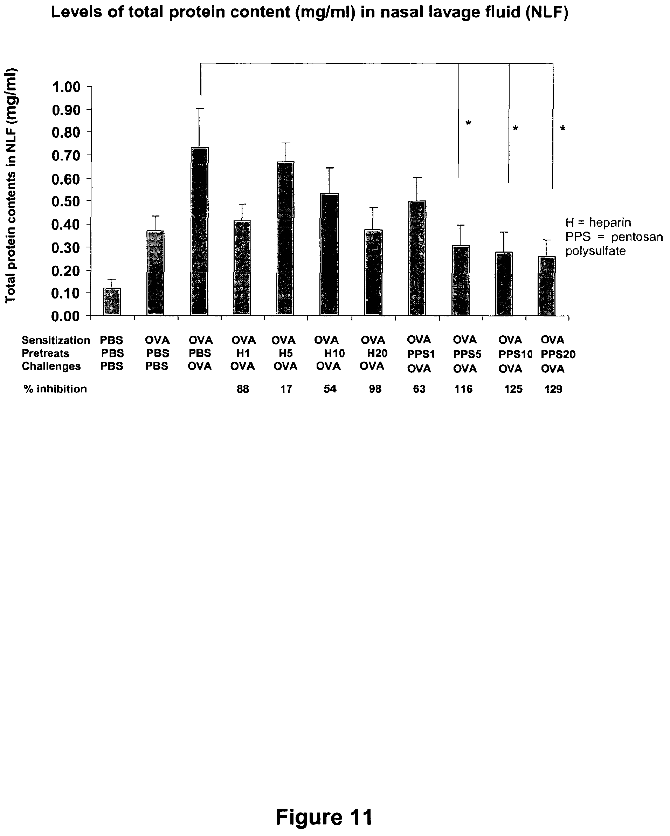

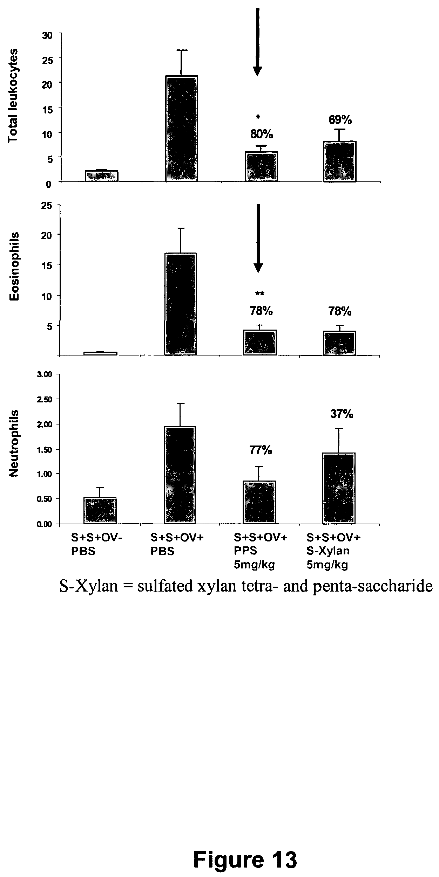

The data indicate that levels of plasma exudation to the nasal cavity were significantly increased 8 hours after intranasal challenge with OVA. PPS significantly reduced the protein content of the nasal lavage fluid indicating a reduction in plasma exudation with this drug (Figure 11). An assessment of leukocyte infiltration into the nasal lavage fluid indicated an increased leukocyte count over that seen with animals sensitized with saline, however PPS inhibited leukocyte infiltration at all the concentrations tested (Figure 12). The most marked leukocytes in the nasal lavage are eosinophils with some evidence of neutrophil infiltration, the levels of other cell types: basophils, lymphocytes, monocytes and nasal epithelial cells are low and not significantly different from that seen in animals sensitized with saline. Animals receiving the corticosteroid Budesonide had a marked decrease in plasma exudation in the nasal lavage and also a marked decrease in the total white cell numbers in the nasal lavage fluid, the most pronounced decrease being in the numbers of eosinophils. PPS similarly markedly reduced the cellular infiltrate into the nasal lavage fluid and like the corticosteroid its most pronounced effect was a reduction in eosinophils (Figure 13). Another sulfated xylan consisting of a mix of tetrasaccharide and pentasaccharide was not as effective an inhibitor of cellular infiltration in to the nasal cavity as PPS (Figure 13).

### EXAMPLE 8

### Pentosan polysulfate is effective in an asthma animal model

A guinea pig model of asthma was used. Guinea-pigs (8 per group) were sensitized to ovalbumin by two intraperitoneal injections of ovalbumin (grade V, 20 µg) in aluminium hydroxide gel on Days 1 and 11. On day 20 (24 hours prior to assessing airway hyper-reactivity (AHR)) each animal received a challenge dose of OVA - this took the form of a 1 hour aerosol exposure of OVA (10 µg). The negative control animals received, no sensitization and no challenge. The positive controls were sensitized and challenged but were either given no further treatment or were treated with vehicle (saline). Test compounds (PPS and the sulfated xylan pentasaccharide) and vehicle were administered by intratracheal administration as an aqueous microspray using a Penn Century device. The device used was the FMJ250 high pressure syringe and a 1A-1C microspray delivery tube. The particle size delivery goes up to 32 µm. During the study the volume delivered was 100 ul for all test formulations. The test compounds were administered 30 min before challenge with OVA. The animals underwent surgery 24 hours after challenge to insert a cannula into the trachea, the cannula was connected to the instrumentation which measured pressure and changes in airflow. After surgery the animals were allowed to stabilize for 10 min before baseline readings were taken. Bronchoconstriction was evoked with aerosolized methacholine (10 mg/ml and 30 mg/ml) for 20 seconds. The difference between baseline readings and that obtained after methacholine were used to calculate C_{dyn} and R_{L}. Thus, measurements of lung function were taken approximately 25 hours after the drug was administered. Both pentosan polysulfate and the sulfated xylan pentasaccharide when administered at 10 mg/kg were effective in reducing the airway hyper-reactivity readings. Their efficacy was approximately equivalent to that usually seen with 7.5 mg/kg of dexamethasone in this model.

**Table 4**

| **AHR data expressed as % decrease from that of the positive control for R_{L} and % increase from that of the positive control for C_{dyn}** | | | | |
|---|---|---|---|---|
| **Compound** | **R_{L}** | | **C_{dyn}** | |
| | MCh 10mg/ml | MCh 30mg/ml | MCh 10 mg/ml | MCh 30 mg/ml |
| Dexamethasone* | 93.9% ** | 86% ** | 76.5%** | 84.8%** |
| Sulfated xylan pentasaccharide^{#} | 34% | 25% * | 48% ** | 37% ** |
| Pentosan polysulfate^{#} | 32% | 35% ** | 38% * | 49% ** |

| | | | | |
|---|---|---|---|---|
| * Dexamethasone 15mg/kg administered by intratracheal microspray # Glycan compounds 10mg/kg administered by intratracheal microspray * Significantly different from positive control P < 0.05 ** Significantly different from positive control P < 0.01 MCh = methacholine | | | | |

A slightly different animal model was also examined. In this model Guinea pigs were sensitized to OVA twice by an intraperitoneal injection of 0.5 ml saline containing 20 mg Al(OH)3 and 20 µg OVA. The sensitizations are performed on days 0 and 7. Three weeks after the last sensitization, animals were pre-treated with either vehicle or drugs 30 min prior to inhalation of OVA (at 10 mg/ml) for 6 min (allergen challenge). For the negative control, animals received either sensitization and challenge with saline or sensitization with saline and challenge with OVA. Animals were terminated and all parameters measured 8 hours after the provocation. Vehicle or drugs were administered intra-tracheally, 1 ml/kg body weight, 30 min before the intra-tracheal challenge with OVA. The vehicle for compounds was saline. Budesonide as the reference compound was dissolved in the vehicle at concentrations of 1 mg/ml. To measure airway resistance (R_{L}.) and lung compliance (C_{dyn}) bronchoconstriction was evoked with aerosolized methacholine (3mg/ml, 10 mg/ml and 30 mg/ml). The difference between baseline readings and that obtained after methacholine were used to calculate C_{dyn} and R_{L}. Bronchoalveolar lavage (BAL) was performed immediately after the lung function measurements. BAL was analysed for protein content (as a measure of leakage) and leukocyte number, differential cell counts were performed to indicate what subsets of leukocytes were best affected by the drugs.

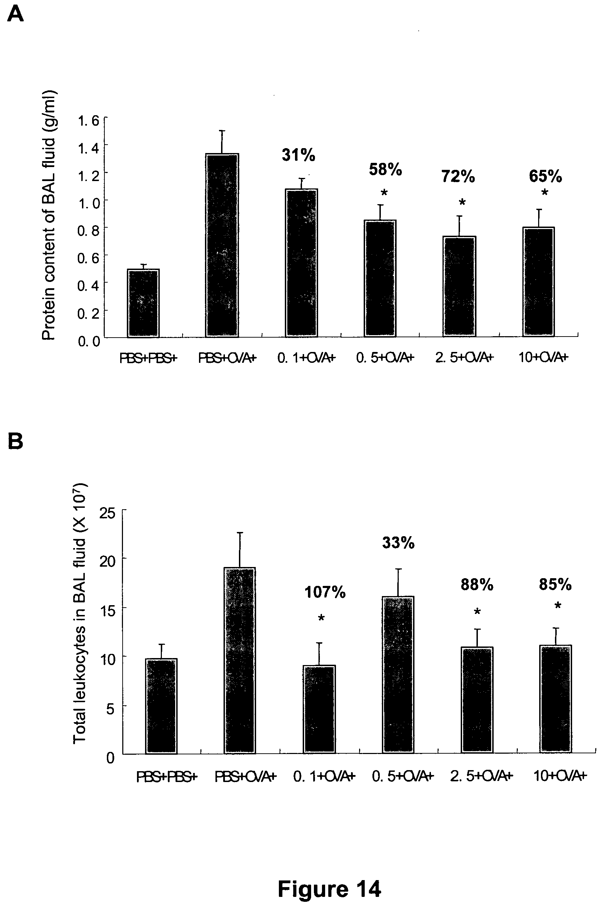

The data indicate that PPS significantly reduced the total number of infiltrating leukocytes in the bronchoalveolar fluid of OVA challenged animals it also significantly reduced the total protein content in the bronchoalveolar fluid. The concentrations tested were 0.1, 0.5, 2.5, and 10 mg/kg body weight. All concentrations of pentosan except 0.1 mg/kg significantly (P<0.05) reduced the protein content from that seen when PBS was administered to OVA sensitized animals (positive control) in a dose dependent manner (Figure 14A). All doses of pentosan also showed inhibitory effects of between 85 and 100% (except for 0.5 mg/kg) on the number of leukocytes infiltrating the respiratory tissues and collected in the bronchoalveolar fluid and the inhibitory effects were significant (P<0.5) (Figure 14B).

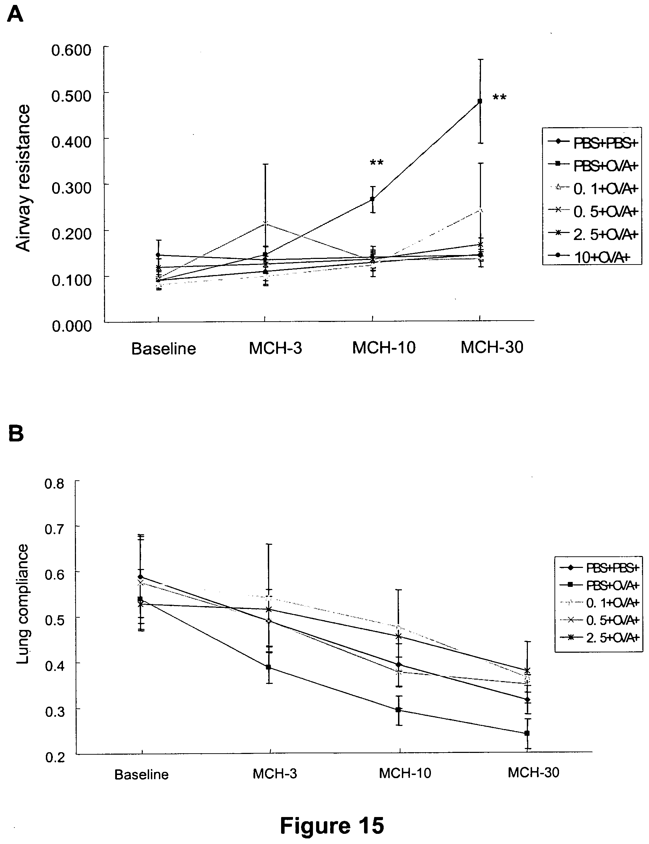

The airway hyperreactivity measurement R_{L} indicated that all doses of pentosan polysulfate showed inhibitory effects of more than 90% after stimulation with 10mg/ml methacholine and the higher doses of 0.5, 2.4 and 10mg/kg of pentosan polysulfate also very significantly inhibited R_{L} (inhibition being 103%, 94% and 100% respectively) (Figure 15A). Inhibitory effects of pentosan polysulfate were also observed on alterations of lung compliance, the three lowest concentrations tested are shown in Figure 15B. These effects are represented as an increase in lung compliance.

### EXAMPLE 9

### Pentosan polysulfate is effective in a COPD animal model

A mouse model of emphysema was used. Acute and chronic exposure of mice to cigarette smoke lead to lung responses that in part mimic the inflammatory and structural changes observed in COPD. In this model male C57B1/6J mice are subjected to acute and chronic smoke exposure, with normal room air being the control situation. In the acute study mice were exposed to either room air or to the smoke of five cigarettes (approximately 12mg of tar and 0.9mg of nicotine) for 20 minutes. In the chronic study mice were exposed to either room air or to the smoke of three cigarettes/day for 5 days/week for 6 months. The 4 groups of mice were then further divided so that mice within each group also received PPS *via* an inhaled route.

In the acute exposure groups of mice pentosan polysulfate was given 30 minutes before exposure to cigarette smoke. Assessment of the efficacy of the drug for mice in the acute exposure groups involved assessment of trolox equivalent anti-oxidant capacity of the bronchoalveolar lavage fluid (BALF) at the end of smoke exposure. The BALF was examined for cytokines and chemokines that are associated with an inflammatory response. The levels of these agents were determined at 4 hours after exposure and at 24 hours after cigarette smoke exposure. A range of cytokines and chemokines was measured. These included: IL-1α, IL-1β, IL-2, IL-3, IL-4, IL-5, IL-6, IL-10, IL-12, RANTES, MIP-1α, cytokine-induced neutrophil chemoattractant (KC), TNF-α and IFN-γ). A differential cell count of the cells in the BALF was also determined. The results of this study indicated that pentosan polysulfate decreased the cellular infiltrate into BALF of animals exposed to cigarette smoke, in particular the numbers of neutrophils were significantly attenuated in those animals exposed to cigarette smoke and which also received PPS.

The animals in the chronic study received PPS once a day for the duration of the experiment. At the completion of the experiment animals were killed and the lungs fixed intratracheally with formalin (5%) and lung volume was measured by water displacement. The lung tissue was prepared for histochemistry and immunohistochemistry, the lung tissues being stained for hematoxylin-eosin and /or periodic acid-Schiff or with antibodies to the macrophage marker Mac-3. The level of desmosine in the lung tissue was also determined. Desmosine is an elastin-specific imino acid; the assessment of desmosine in the lung is taken as an indicator of the lung elastin content. A decline in desmosine content is evidence that the emphysematous changes are associated with proteolysis and matrix breakdown. Collectively the results of this study indicated PPS is acting as an anti-inflammatory agent in this COPD model.

### EXAMPLE 10

### Pentosan polysulfate and a DP5 sulfated xylan inhibit the activity of the asthma protein IL-13

IL-13 is a cytokine with a key role in asthma and allergic disease (including allergic rhinitis) and it seems particularly important in blocking the airway hyperreactivity seen with asthma (Wills-Karp, Immunol. Rev. 202:175-190, 2004). Recent data suggest that IL-13 also has a very important role in COPD (Tyner et al, J. Clin. Invest. 116:309-321, 2006; Lee et al, Respir. Res. 8:64-73, 2007). Pentosan polysulfate (PPS) and a DP5 sulfated xylan inhibited the proliferation of a human IL-13 responsive cell line. This occurs at very low doses and is not due to a toxic effect of the PPS because other, similarly sulfated polysaccharides, at the same concentrations of IL-13 and polysaccharide have no effect. These experiments utilize the TF-1 cells. TF-1 cells were originally established from a bone marrow sample from a male with severe pancytopenia. These cells are dependent on IL-3 or GM-CSF for long term growth and are responsive to a variety of cytokines including IL-4 and IL-13.

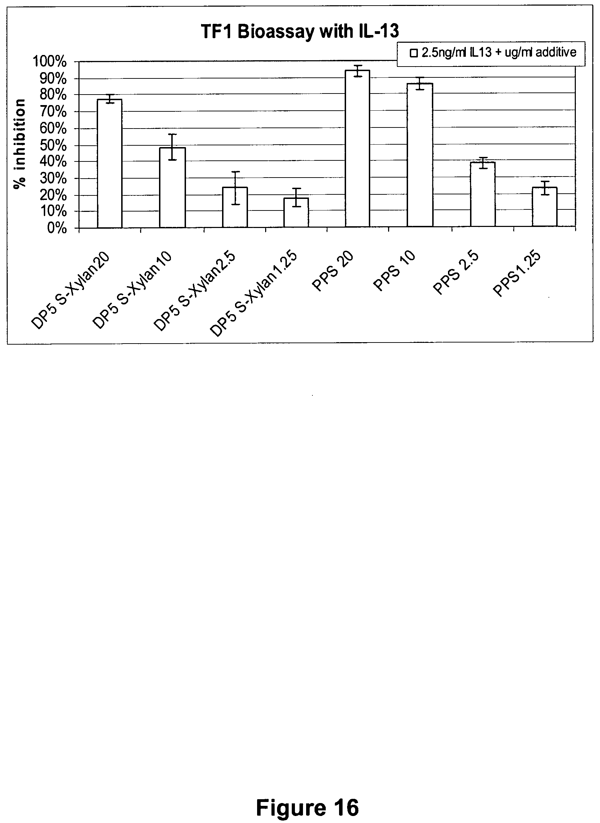

The proliferation assays are carried out in 96-well microplates suitable for such assays (Falcon). The wells are flat bottomed, with white sides and a clear bottom. Cells are washed to remove any cytokine in the growth medium and then resuspended in RPMI/5% w/v FCS. The cells are counted with a Coulter Z2 Particle Counter and Size Analyzer (Coulter Electronics, England) and routinely 2.5 x 10⁴ cells are added to microplate wells that contain either no IL-13 (negative control) or various dilutions of IL-13. When the effect of PPS is to be measured, the wells also contain various concentrations of this molecule. The cells proliferate for 48 hours at 37°C in a humidified atmosphere, after which the number of cells present is quantified by staining with the AQUEOUS ONE dye (30µl/well) for 3 hours and then absorbance is read at 490 nm. Using this assay, the inventors demonstrated that PPS and a DP5 sulfated xylan markedly inhibit the IL-13 dependent proliferation of TF-1 cells (Figure 16) with 75-80% of IL-13 mediated cell proliferation blocked at PPS concentrations of 5-10 µg/ml.

### EXAMPLE 11

### Pentosan polysulfate and a DP5 sulfated xylan inhibit the activity of various chemokines that play a role in inflammation associated with COPD

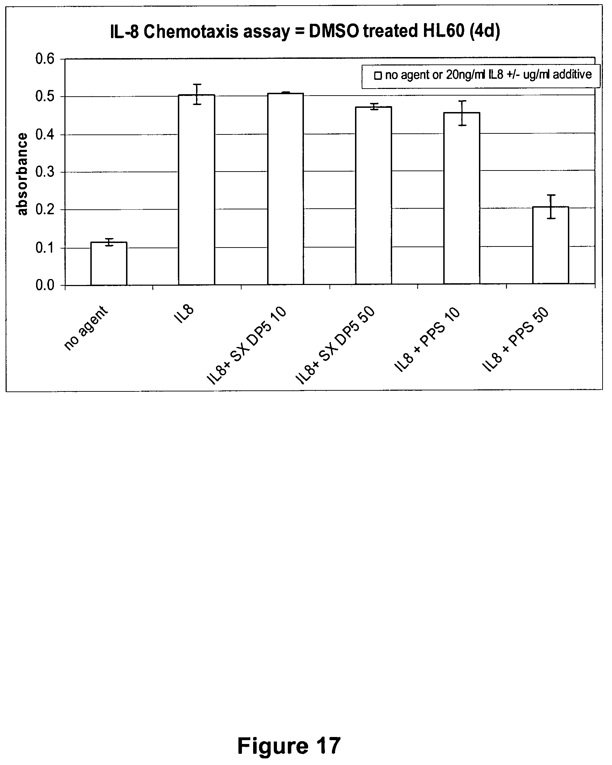

Chemokines known to play an important role in mediating the inflammation associated with COPD include IL-8, MCP-1 and MIP-1α (Barnes 2004, *supra*). PPS was shown to block cell migration triggered by IL-8. These experiments were performed using DMSO treated human promyelocytic HL-60 cells. These cells were derived from a patient with acute promyelocytic leukemia. The cells were treated with DMSO (1.2%) for 4 days before being used in the experiments. The chemotaxis assays were performed in 96-well Costar chemotaxis plates consisting of a bottom chamber to which was added the human IL-8 (+/- inhibitor) and then cells in RPMI and 1% v/v FCS were added to a top chamber and the plate was incubated at 37° C for 1 hour to allow cells to move from the top chamber into the bottom. The number of cells migrating into the bottom chamber was quantified by labeling with AQUEOUS ONE (20µl/well) for 1.75 hours before absorbance at 490 nm is read. IL-8 was used at a final concentration of 20ng/ml and the inhibitors were the sulfated xylan pentasaccharide (10 and 50µg/ml) and pentosan polysulfate (10 and 50µg/ml). Cell migration is compared with that seen with no IL-8 or with IL-8 and no inhibitor. These data indicate that PPS is a good inhibitor in this assay as cell migration is inhibited by 74% with 50µg/ml of PPS (Figure 17).

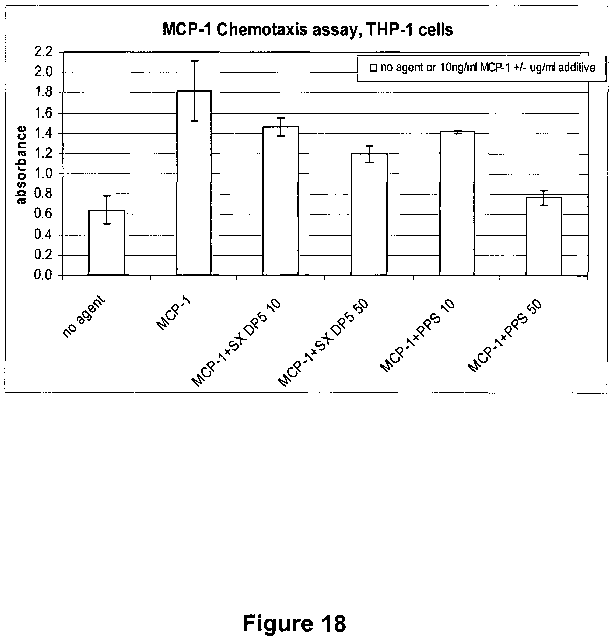

To examine whether PPS or the sulfated xylan pentasaccharide were effective inhibitors of the chemokine MCP-1 the human monocytic cell line THP-1 was used. These cells were originally derived from the peripheral blood of a patient with acute monocytic leukaemia. The assay was very similar to that described above. The chemotaxis assays were performed in 96-well Costar chemotaxis plates and human MCP-1 (+/- inhibitor) was added to the bottom chamber and the THP-1 cells in RPMI/1% FCS were added to the top chamber and the plate was incubated at 37° C for 2.5 hour to allow cells to move from the top chamber into the bottom. The number of cells migrating into the bottom chamber was quantified by labeling with AQUEOUS ONE (30µl/well) for 4 hours before absorbance at 490 nm is read. MCP-1 was used at a final concentration of 10ng/ml and the inhibitors were the sulfated xylan pentasaccharide (10 and 50µg/ml) and PPS (10 and 50µg/ml). In these experiments both PPS and the sulfated xylan pentasaccharide were effective, but PPS was a better inhibitor (Figure 18) as at 50 µg/ml PPS inhibited cell movement by 89% whereas at the same concentration the sulfated xylan pentasaccharide cell movement by 53%.

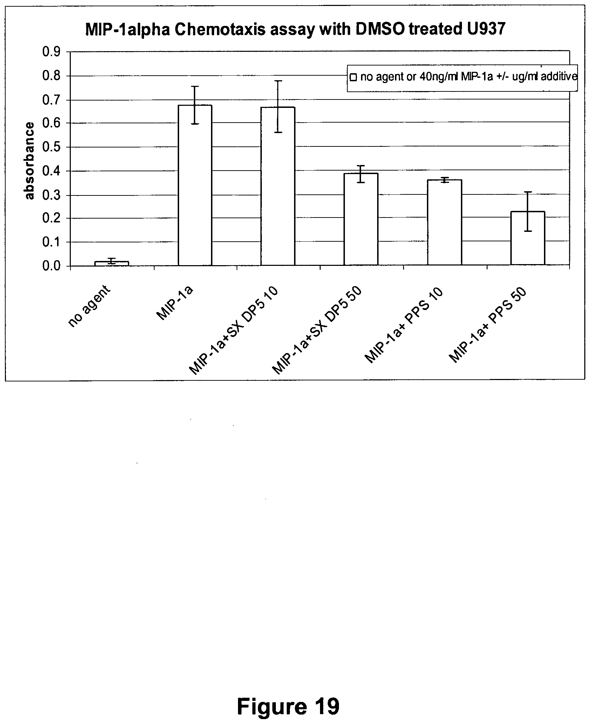

DMSO treated U937 cells were used to examine the cell migration in response to MIP-1α. U937 cells are a promonocytic human cell line originally derived from the pleural effusion of a patient with histiocytic lymphoma. These cells were treated with DMSO (1.2%) for 4 days before being used in chemotaxis experiments. The chemotaxis assays were performed in 96-well Costar chemotaxis plates and human MIP-1α (+/- inhibitor) was added to the bottom chamber and the DMSO treated U937 cells in RPMI/HEPES were added to the top chamber and the plate was incubated at 37° C for 3 hour to allow cells to move from the top chamber into the bottom. The number of cells migrating into the bottom chamber was quantified by labeling with AQUEOUS ONE (30µl/well) for 1 hour before absorbance at 490 nm is read. MIP-1α was used at a final concentration of 40ng/ml and the inhibitors were the sulfated xylan pentasaccharide (10 and 50µg/ml) and PPS (10 and 50µg/ml). It is clear from these experiments that both PPS and the sulfated xylan pentasaccharide inhibited MIP-1α stimulated cell movement. PPS was more effective being a good inhibitor at both 10 and 50µg/ml, inhibiting cell movement by respectively 48% and 69%; where as the sulfated xylan pentasaccharide was only effective at 50µg/ml, inhibiting cell movement by 44% (Figure 19).

These data indicate that PPS inhibits cell movement induced by three key chemokines IL-8, MCP-1 and MIP-1α known to play a role in the inflammations associated with COPD.

Those skilled in the art will appreciate that the invention described herein is defined by the claims, and disclosed by the specification. The disclosure also includes all of the steps, features, compositions and compounds referred to or indicated in this specification, individually or collectively, and any and all combinations of any two or more of said steps or features.

### BIBLIOGRAPHY

Adcock and Ito, Proc. Am. Thorac. Soc. 2:313-319, 2005
Allen, Adv Pediatr. 53:101-110, 2006
Anzueto, Am. J. Med. 119:S46-S53, 2006
Barnes, Pharmacol. Rev. 56:515-548, 2004
Barnes, .Br. J. Pharmacol. 147 Suppl 1:S297-303, 2006
Barnes and Stockley, Eur. Respir. J. 25:1084-1106, 2005
Bayol et al, US Patent 4,713,373
Blaiss, Allergy Asthma Proc. 26: 35-40, 2005
Borish, J. Allergy Clin Immunol. 112: 1021-1031, 2003
Casu, Adv. Carbohydr. Chem. Biochem. 43: 51-134, 1985
Casu, Ann. N.Y. Acad. Sci. 556: 1-17, 1989
Coombe et al, Journal of Immunological Methods 215: 145-150, 1998
Degenhardtet al, Arch Pharm (Weinheim) 334(1):27-9, 2001
Elmiron".Pentosan Polysulfate Sodium. Product Monograph.Janssen-Ortho Inc January 2006
Fernig, In: Proteoglycan protocols, Ed. R.V. Iozzo, Humana Press, Totowa, NJ, USA, p. 505-518, 2001
Gelfand, J. Allergy Clin. Immunol 114: S135-138, 2004
Golightly and Greos. Drugs 65:341-384 2005
Green et al, Curr. Opin. Allergy Immunol. 7:43-50, 2007
Halpin and Miravitlles. Proc. Am. Thorac. Soc. 3:619-623, 2006
Kuszmann et al, Patent WO 2006017752
Lander and Selleck, J. Cell Biol. 148(2): 227-232, 2000
Lee et al, Respir. Res. 8:64-73, 2007
MacNee, Proc. Am. Thorac. Soc. 2:258-266, 2005
Neilsen and Dahl, Am. J. Respir. Med. 2:55-65, 2003
Palmqvist et al., Allergy 60:65. 2005
Parish et al, US Patent 6,143,730
Passalacqua et al, Curr. Opin. Allergy Clin. Immunol. 4:177-183. 2004
Pawanker, Curr. Opin. Allergy Clin. Immunol. 4:1-4. 2004
Sasisekharan and Venkataraman, Curr. Opin. Chem. Biol. 4(6): 626-631, 2000
Skoner, Curr. Opin. Allergy Clin. Immunol. 2:7-10. 2002.
Suchankova et al, Eur J Pharmacol. 507(1-3):261-71, 2005
Sutherland and Martin, JAllergy Clin. Immunol. 112:819-27. 2003
Szilasi et al, Pathol. Oncol. Res. 12:52-60. 2006
Tyner et al, J. Clin. Invest. 116:309-321, 2006
Venkataraman et al, Science 286(5439):537-542, 1999
Walls et al, Med. J. Aust. 182:28-33, 2005
Wenzel, Lancet 368:804-813, 2006
Wills-Karp, Immunol. Rev. 202:175-190, 2004
Yanez and Rodrigo, Ann. Allergy Asthma Immunol. 89:479-84. 2002

## Claims

1. Pharmaceutical composition comprising an effective amount of a sulfated xylan, wherein the sulfated xylan is pentosan polysulfate (PPS), for use in the treatment or prophylaxis of an inflammatory respiratory condition selected from the list consisting of asthma, allergic rhinitis and chronic obstructive pulmonary disease (COPD) in a subject.

2. The pharmaceutical composition of Claim 1 wherein the subject is a human.

3. The pharmaceutical composition of Claim 2 further comprising an anti-inflammatory agent.

4. The pharmaceutical composition of Claim 3 wherein the anti-inflammatory agent is an anti-histamine, an antagonist of G-CSF, M-CSF and/or GM-CSF, an antagonist of IL-5, IL-4 or IL-13, an antagonist of eotaxin-1 or eotaxin-2 or an antagonist of IL-8, MCP-1 or MIP-1α.

5. Use of a sulfated xylan, wherein the sulfated xylan is PPS, in the manufacture of a medicament for the treatment or prophylaxis of an inflammatory respiratory condition selected from the list consisting of asthma, allergic rhinitis and chronic obstructive pulmonary disease (COPD) in a subject.

6. Use of any one of Claim 5 wherein the subject is a human.

7. Use of Claim 6 further comprising the use of an anti-inflammatory agent.

8. Use of Claim 7 wherein the anti-inflammatory agent is an anti-histamine, an antagonist of G-CSF, M-CSF and/or GM-CSF, an antagonist of IL-4 or IL-5 or IL-13, an antagonist of eotaxin-1 or eotaxin-2 or an antagonist of IL-8 or MCP-1, or MIP-1α.

9. Pharmaceutical composition comprising an effective amount of PPS and an anti-inflammatory agent selected from the list consisting of an antagonist of G-CSF, MCSF, GM-CSF, IL-4, IL-5, IL-13, eotaxin-1, eotaxin-2, MCP-1 and MIP-1α for use in the treatment of a subject with asthma, allergic rhinitis or COPD.

## Patentansprüche

1. Pharmazeutische Zusammensetzung, umfassend eine wirksame Menge an sulfatiertem Xylan, wobei das sulfatierte Xylan Pentosanpolysulfat (PPS) ist, zur Verwendung bei der Behandlung oder Prophylaxe einer entzündlichen Atemwegserkrankung, ausgewählt aus der Liste bestehend aus Asthma, allergischer Rhinitis und chronisch obstruktiver Lungenerkrankung (COPD), bei einem Individuum.

2. Pharmazeutische Zusammensetzung gemäss Anspruch 1, wobei das Individuum ein Mensch ist.

3. Pharmazeutische Zusammensetzung gemäss Anspruch 2, die ferner ein entzündungshemmendes Mittel umfasst.

4. Pharmazeutische Zusammensetzung gemäss Anspruch 3, wobei das entzündungshemmende Mittel ein Antihistamin, ein Antagonist von G-CSF, M-CSF und/oder GM-CSF, ein Antagonist von IL-5, IL-4 oder IL-13, ein Antagonist von Eotaxin-1 oder Eotaxin-2 oder ein Antagonist von IL-8, MCP-1 oder MIP-1α ist.

5. Verwendung eines sulfatierten Xylans, wobei das sulfatierte Xylan PPS ist, bei der Herstellung eines Arzneimittels zur Behandlung oder Prophylaxe einer entzündlichen Atemwegserkrankung, ausgewählt aus der Liste bestehend aus Asthma, allergischer Rhinitis und chronisch obstruktiver Lungenerkrankung (COPD), bei einem Individuum.

6. Verwendung gemäss Anspruch 5, wobei das Individuum ein Mensch ist.

7. Verwendung gemäss Anspruch 6, die ferner die Verwendung eines entzündungshemmenden Mittels umfasst.

8. Verwendung gemäss Anspruch 7, wobei das
entzündungshemmende Mittel ein Antihistamin, ein Antagonist von G-CSF, M-CSF und/oder GM-CSF, ein Antagonist von IL-4 oder IL-5 oder IL-13, ein Antagonist von Eotaxin-1 oder Eotaxin-2 oder ein Antagonist von IL-8 oder MCP-1 oder MIP-1α ist.

9. Pharmazeutische Zusammensetzung, umfassend eine wirksame Menge an PPS und einem entzündungshemmenden Mittel, ausgewählt aus der Liste bestehend aus einem Antagonisten von G-CSF, M-CSF, GM-CSF, IL-4, IL-5, IL-13, Eotaxin-1, Eotaxin-2, MCP-1 und MIP-1α, zur Verwendung bei der Behandlung eines Individuums, das an Asthma, allergischer Rhinitis oder COPD leidet.

## Revendications

1. Composition pharmaceutique comprenant une quantité efficace d'un xylane sulfaté, où le xylane sulfaté est le polysulfate de pentosane (PPS), pour une utilisation dans le traitement ou la prophylaxie d'une affection respiratoire inflammatoire choisie dans la liste constituée de l'asthme, de la rhinite allergique et de la bronchopneumopathie chronique obstructive (BPCO) chez un sujet.

2. Composition pharmaceutique selon la revendication 1, où le sujet est un être humain.

3. Composition pharmaceutique selon la revendication 2 comprenant en outre un agent anti-inflammatoire.

4. Composition pharmaceutique selon la revendication 3, où l'agent anti-inflammatoire est un antihistaminique, un antagoniste du G-CSF, du M-CSF et/ou du GM-CSF, un antagoniste de l'IL-5, de l'IL-4 ou de l'IL-13, un antagoniste de l'éotaxine 1 ou de l'éotaxine 2 ou un antagoniste de l'IL-8, de la MCP-1 ou de la MIP-1α.

5. Utilisation d'un xylane sulfaté, où le xylane sulfaté est le PPS, dans la fabrication d'un médicament pour le traitement ou la prophylaxie d'une affection respiratoire inflammatoire choisie dans la liste constituée de l'asthme, de la rhinite allergique et de la bronchopneumopathie chronique obstructive (BPCO) chez un sujet.

6. Utilisation selon l'une quelconque de la revendication 5, où le sujet est un être humain.

7. Utilisation selon la revendication 6 comprenant en outre l'utilisation d'un agent anti-inflammatoire.

8. Utilisation selon la revendication 7 où l'agent anti-inflammatoire est un antihistaminique, un antagoniste du G-CSF, du M-CSF et/ou du GM-CSF, un antagoniste de l'IL-4 ou de l'IL-5 ou de l'IL-13, un antagoniste de l'éotaxine 1 ou de l'éotaxine 2 ou un antagoniste de l'IL-8 ou de la MCP-1 ou de la MIP-1α.

9. Composition pharmaceutique comprenant une quantité efficace de PPS et d'un agent anti-inflammatoire choisi dans la liste constituée d'un antagoniste du G-CSF, du M-CSF, du GM-CSF, de l'IL-4, de l'IL-5, de l'IL-13, de l'éotaxine 1, de l'éotaxine 2, de la MCP-1 et de la MIP-1α pour une utilisation dans le traitement d'un sujet souffrant d'asthme, de rhinite allergique ou de BPCO.
